# EUROPEAN PATENT SPECIFICATION

(11) **EP 0 804 219 B1**
(45) Date of publication and mention of the grant of the patent: **17.07.2002**
(21) Application number: 95913576.5
(22) Date of filing: 28.02.1995
(51) Int. Cl.: A61K 38/04, A61K 38/05, A61K 38/06, A61K 38/07, A61K 38/08, A61K 38/10, A61K 38/16, C07K 9/00, C07K 14/00, C07K 17/00, A61K 39/385, C07K 14/575

(54) **INHIBIN COMPOSITIONS AND METHODS OF USE THEREOF**
Inhibin enthaltende Zusammensetzung und ihre Verwendung
COMPOSITIONS A BASE D'INHIBINE ET APPLICATIONS

(30) Priority: 28.02.1994 US 202964
(43) Date of publication of application: 05.11.1997
(73) Proprietor: Agritech Technologies, Ltd., Irving, Texas 75038 (US); THE BOARD OF SUPERVISORS OF LOUISIANA STATE UNIVERSITY AND AGRICULTURAL AND MECHANICAL COLLEGE, Baton Rouge, LA 70803 (US)
(72) Inventor: FIORETTI, William, C., Grapevine, TX 76051 (US); KOUSOULAS, Konstantin, Baton Rouge, LA 70810 (US); SATTERLEE, Daniel, G., Prairieville, LA 70769 (US)
(74) Representative: Fleischer, Holm Herbert, Dr.
(86) International application number: US9502795
(87) International publication number: WO9522980

(56) References cited:
- US-A- 4 864 019
- US-A- 5 089 396
- CHOULJENKO ET AL.: "Expression and purification of chicken alpha-inhibin as a fusion protein with the E. coli maltose binding protein" POULTRY SCIENCE, vol. 73, no. sup1, 1994, page 84 XP002088124
- WANG ET AL.: "Complementary deoxyribonucleic acid cloning and sequence analysis of the alpha-subunit of inhibin from chicken ovarian granulosa cells" BIOLOGY OF REPRODUCTION, vol. 49, 15 June 1993, pages 1-6, XP002088125
- JOURNAL OF REPRODUCTION AND FERTILITY, Volume 95, issued 1992, J.H.M. WRATHALL et al., "Effects of Active Immunization Against a Synthetic Peptide Sequence of the Inhibin alpha-Subunit on Plasma Gonadotropin Concentrations, Ovulation Rate and Lambing Rate in Ewes", pages 175-182.
- BIOLOGY OF REPRODUCTION, Volume 49, issued 1993, S.Y. WANG et al., "Complementary Deoxyribonucleic Acid Cloning and Sequence Analysis of the alpha-Subunit of Inhibin from Chicken Ovarian Granulosa Cells", pages 1-6.
- JOURNAL OF ANIMAL SCIENCE, Volume 69, issued 1991, R.L. MEYER et al., "Antiserum to an Inhibin Alpha-Chain Peptide Neutralizes Inhibin Bioactivity and Increases Ovulation Rate in Sheep", pages 747-754.
- JOURNAL OF ENDOCRINOLOGY, Volume 134, issued 1992, R.G. GLENCROSS et al., "Effect of Active Immunization of Heifers Against Inhibin on Plasma FSH Concentrations, Ovarian Follicular Development and Ovulation Rate", pages 11-18.
- ENDOCRINOLOGY, Volume 123, issued 1988, C. RIVIER et al., "Age-dependent Changes in Physiological Action, Content and Immunostaining of Inhibin in Male Rats", pages 120-126.
- JOURNAL OF REPRODUCTION AND FERTILITY, Volume 97, issued 1993, D.G. MORRIS et al., "Effect of Immunization Against Synthetic Peptide Sequences of Bovine Inhibin alpha-Subunit on Ovulation Rate and Twin-calving Rate in Heifers", pages 255-261.
- JOURNAL OF REPRODUCTION AND FERTILITY, Volume 97, issued 1993, A.R. SCANLON et al., "Active Immunization of Heifers Against a Synthetic Fragment of Bovine Inhibin", pages 213-222.

### Sequence Listing

(1) GENERAL INFORMATION:
   (i) APPLICANTS: Fioretti, William C.
      Kousoulas, Konstantin
      Satterlee, Daniel G.
   (ii) TITLE OF INVENTION: Inhibin Compositions and Methods of
      Use Thereof
   (iii) NUMBER OF SEQUENCES: 4
   (iv) CORRESPONDENCE ADDRESS:
      (A) ADDRESSEE: Jones & Askew
      (B) STREET: 191 Peachtree Street, 37th Floor
      (C) CITY: Atlanta
      (D) STATE: Georgia
      (E) COUNTRY: USA
      (F) ZIP: 30303-1769
   (v) COMPUTER READABLE FORM:
      (A) MEDIUM TYPE: Diskette, 3.50
      (B) COMPUTER: MacIntosh
      (C) OPERATING SYSTEM: 7.0
      (D) SOFTWARE: Microsoft Word
   (vi) CURRENT APPLICATION DATA:
      (A) APPLICATION NUMBER:
      (B) FILING DATE:
      (C) CLASSIFICATION:
         (vii) PRIOR APPLICATION DATA:

      (A) APPLICATION NUMBER: 08/202,964
      (B) FILING DATE: February 28, 1994
   (viii) ATTORNEY/AGENT INFORMATION:
      (A) NAME: Roxanne Edwards Cenatempo
      (B) REGISTRATION: 38, 767
      (C) REFERENCE/DOCKET NUMBER: 01051-0111
   (ix) TELECOMMUNICATION INFORMATION:
      (A) TELEPHONE: 404-818-3700
      (B) TELEFAX: 404-818-3799
(2) INFORMATION FOR SEQ ID NO: 1:
   (i) SEQUENCE CHARACTERISTICS
      (A) LENGTH: 515 base pairs
      (B) TYPE: Nucleic Acid
      (C) STRANDEDNESS: Single
      (D) TOPOLOGY: Linear
   (xi) SEQUENCE DESCRIPTION:
(3) INFORMATION FOR SEQ ID NO: 2:
   (i) SEQUENCE CHARACTERISTICS
      (A) LENGTH: 99 residues
      (B) TYPE: Amino Acid
      (C) STRANDEDNESS:
      (D) TOPOLOGY: Linear
   (xi) SEQUENCE DESCRIPTION:
   Arg Pro Ser Glu Asp Val Ala Ala His Thr Asn Cys Arg Arg Ala        15
   Ser Leu Asn Ile Ser Phe Glu Glu Leu Gly Trp Asp Asn Trp Ile Val    31
   His Pro Ser Ser Phe Val Phe His Tyr Cys His Gly Asn Cys Ala Glu    47
   Gly Asp Gly Leu Ser His Arg Leu Gly Val Gln Leu Cys Cys Ala Ala    63
   Leu Pro Gly Thr Met Arg Ser Leu Arg Val Arg Thr Thr Ser Asp Gly    79
   Gly Tyr Ser Phe Lys Tyr Glu Thr Val Pro Asn Ile Leu Ala Gln Asp    95
   Cys Thr Cys Val                                                    99
(4) INFORMATION FOR SEQ ID NO: 3:
   (i) SEQUENCE CHARACTERISTICS
      (A) LENGTH: 516 base pairs
      (B) TYPE: Nucleic Acid
      (C) STRANDEDNESS: Single
      (D) TOPOLOGY: Linear
   (xi) SEQUENCE DESCRIPTION:
   G CGC CCA TCG GAG GAC GTG GCC GCC CAC ACC AAC TGC CGC CGG GCG       46
   TCC CTC AAC ATC TCT TTC GAG GAG CTG GGC TGG GAC AAT TGG ATC GTG     94
   CAC CCC AGC AGC TTC GTT TTC CAC TAC TGC CAC GGG AAC TGT GCC GAA    142
   TGG CCA CGG GCT GAG CCA CCG GCT GGG GGT GCA GCT GTG CTG CGC CGC    190
   GCT GCC CGG CAC CAT GCG CTC ACT GCG TGT CCG CAC CAC CTC TGA        235
   TGGTGGCTAC TCCTTCAAGT ACGAGACGGT GCCCAACATC CTGGCGCAGG             285
   ACTGCACCTG TGTCTAGCAG CTGGCATGCA CGGCCAGACC CGCGTGGATC             335
   TCCCCGTTGC CTCTGGACTG CCCCAGTGCC AGATGATGAG CCCATCCCAG             385
   GGATGGAGGA GTCACTCACA CGGGCACTGC GCAGCCCGGA GCAGGGAGAG             435
   GGACCCAGGT GGAAGTTTTG GTGGTGCCAC CCTCCCTTTG ACTGCCAGGG             485
   TTTCATGGTT TCAGGTTGCG TGGGTGCTGC A                                 516
(5) INFORMATION FOR SEQ ID NO: 4:
   (i) SEQUENCE CHARACTERISTICS
      (A) LENGTH: 77
      (B) TYPE: Amino Acid
      (C) STRANDEDNESS:
      (D) TOPOLOGY: Linear
   (xi) SEQUENCE DESCRIPTION:
   Arg Pro Ser Glu Asp Val Ala Ala His Thr Asn Cys Arg Arg Ala        15
   Ser Leu Asn Ile Ser Phe Glu Glu Leu Gly Trp Asp Asn Trp Ile Val    31
   His Pro Ser Ser Phe Val Phe His Tyr Cys His Gly Asn Cys Ala Glu    47
   Trp Pro Arg Ala Glu Pro Pro Ala Gly Gly Ala Ala Val Leu Arg Arg        63
   Ala Ala Arg His His Ala Leu Thr Ala Cys Pro His His Leu                77

## Description

### Field of the Invention

The present invention relates, in general, to a method of accelerating the onset of puberty in avians, more particularly in ratites and psittaciformes, by administering a heterologous protein comprised of alpha-subunit avian inhibin protein, or a fragment thereof, and a carrier protein. The present invention also relates to a method of increasing egg production in birds, and more particularly in ratites, by administering a heterologous protein comprised of alpha-subunit avian inhibin protein, or a fragment thereof, and a carrier protein. The present invention further relates to a heterologous protein comprised of alpha-subunit avian inhibin protein, or a fragment thereof, and a carrier protein, and a method of producing the heterologous protein, wherein the inhibin is fused to the carrier protein.

### Background of the Invention

Ratites are flightless, generally large, running birds, comprising several orders including the species Ostrich, Emu, Rhea, Cassowaries, and Kiwis. An emu (*Dromiceius novaehollandiae*) is an Australian ratite bird which is characterized by rudimentary wings and a feathered head and neck. An average adult emu is approximately 6 feet tall and weighs approximately 150 pounds. An ostrich (*Struthio camelius*) is a large running bird with small wings and thick powerful legs. A standard adult ostrich is approximately 8 feet tall and weighs approximately 325 to 375 pounds. The term "rhea" is the common name for members of the avian order Rheiformes. Rheiformes are an order of South American running birds, called American ostriches, which differ from the true ostrich in their smaller size, feathered head and neck, and three-toed feet among other features.

The ostrich and emu have long had a commercial value in their natural environments of South Africa and Australia, respectively. Ostrich products have been in demand for more than 100 years, and a substantial worldwide market exists for their hides, meat, and feathers. For example, ostrich leather is used in boots, handbags, jackets, attache cases, wallets, and many other articles. Ostrich feathers are used in fashion, costuming, and in feather dusters. For example, ostrich feathers are sold in Europe for approximately $60 to $1,200 per kilogram of feathers. Also, ostrich feathers are considered to be "dust magnets" and are therefore in high demand in computer and automotive industries in the United States and abroad.

In contrast, the emu is a relative newcomer to the market place. It is valued for the same products with the addition of an essential oil that is used in the cosmetic industry. Emu oil, rendered from a thick layer of subcutaneous fat has deep penetrating properties which make the oil useful in cosmetic creams, such as, wrinkle retardant emollients. Also, possible medicinal uses for emu oil, such as the treatment of arthritis, are currently being investigated. A typical full-grown emu can achieve a height of 1.6 to 1.9 m or more, and weight 30 to 45 kg or more. Emus mature at about one year, and pre- and post-pubescent emus never show gender specific phenotypic differences. Similar to the ostrich, the emu population in the United States has also experienced explosive growth within the last several years. As of 1994, there were approximately 150,000 total emu, including 15,000 breeding pairs, in the United States. It is predicted that the numbers of emus in 1995 will further increase to between 500,000 and 750,000 birds, of which 45,000 are expected to be breeding pairs.

There is a growing demand for ratite products in several countries, including Australia, Belgium, Israel, Canada, the Netherlands, Namibia, South Africa, and Zimbabwe. Accordingly, over the past several years there has been explosive growth in the domestic market for ostrich and emu, and to a lesser extent rhea. In the last five years, the number of breeding ostrich pairs and total bird numbers in the United States has increased 7.5- and 20-fold, respectively. It is estimated that in 1995, 200,000 ostrich, including 20,000 breeding pairs, will exist in the United States. The tremendous interest in breeding these animals is due to the significant value of adults, as well as immature animals, and especially for proven breeding pairs of ostrich which are valued as high as $75,000.00, with an emu pair valued at $30,000.00 or more. Immature ostriches at three to four months of age are valued at approximately $7,500.00, and immature emu are valued at approximately $5,000.00. A majority of the animals are purchased between three and six months of age.

Further, there is tremendous interest in ratites as an alternative to more traditional forms of animal agriculture. Several factors relating to ratites make them a superior alternative to the more traditional forms of animal agriculture (i.e., beef cattle, swine and sheep farming). These factors include: superior feed conversion ratios, a greater ability to be intensively farmed, large animal size, enhanced reproductive capacity, and exceptional nutritional value of their meat.

For example, ostrich meat, which is a red meat resembling beef, contains significantly less fat, calories, and cholesterol than chicken or turkey meat. More particularly, an 85 gram portion of Ostrich meat contains 2 grams of fat, 58 mg of cholesterol, and 97 calories. In contrast, an 85 gram portion of turkey meat contains 3 grams of fat, 59 mg of cholesterol, and 135 calories. An 86 gram portion of chicken meat contains 3 grams of fat, 73 mg of cholesterol, and 140 calories. An 85 gram portion of beef (steak) contains 15 grams of fat, 77 mg of cholesterol, and 240 calories. And finally, an 85 gram portion of pork contains 19 grams of fat, 84 mg of cholesterol, and 275 calories. (Values for ostrich meat were derived from AMSI Quality Laboratory Report # 0800100. Values for the other meats were derived from U.S.D.A. Handbook No. 8, "Nutritive Value of Foods".) Similar to the ostrich, emu meat is a low fat red meat. More particularly, a 100 gram portion of emu meat contains 1.7 g fat, 57.5 mg cholesterol, and 109 calories. (Values for emu meat were derived from Silliker Laboratories of Texas, Inc.)

Also, ratites such as ostriches provide approximately 100 pounds of meat at the age of 12 months and therefore produce a substantial amount of meat in a relatively short period of time.

An illustration of how ratites are a superior alternative to the more traditional forms of animal agriculture is the following comparison of an ostrich and a cow. First, an ostrich has a gestation and incubation period of 42 days, wherein a cow requires 280 days. Second, an average ostrich produces more than 20 offspring per year, whereas a cow produces one offspring per year. Third, the feed conversion ratio of an ostrich is less than 2:1 whereas the feed conversion ratio of a cow is 5:1. Fourth, the days from conception to slaughter is approximately 407 days for an ostrich in contrast to 645 for a cow. Finally, ostriches produce feathers in addition to their meat and leather, whereas cows do not produce products other than meat and leather.

Considering these attributes, and the increasing needs of the world population for meat which is nutritious yet low in fat and cholesterol and which may be efficiently produced with a minimum negative impact on the environment, the ratite industry has a high potential for future growth.

Currently, the demand for ratites far outweighs the supply. However, ratite producers are limited by sub-optimal egg production in most breeding age females. Depending on the species, most ratites in captivity currently lay an average of 10-20 eggs per year whereas their genetic potential for egg production is thought to exceed over 60 eggs per year. For example, a high-producing ostrich in the wild can lay an egg approximately every 48 hours during breeding season, and a high producing emu in the wild can lay an egg approximately every 72 hours in breeding season. In contrast, an ostrich in captivity often takes 5 to 10 days to lay an egg, and an emu often takes 4 to 8 days to lay an egg.

A slaughter market of ratites will not evolve until sufficient numbers of offspring are produced annually. According to some estimates, in order for a slaughter market to be maintained, there must be at least 250,000 animals available annually. Therefore, a method of enhancing egg production will greatly accelerate the growth of the market. Accordingly, there is a need for a composition and method of increasing egg production in birds, and particularly in ratites such as ostriches and emus.

There also is a need for a composition and method of increasing egg production in exotic birds, such as the Psittaciformes. Psittaciformes include parrots, and are a monofamilial order of birds that exhibit zygodactylism and have a strong hooked bill. A parrot is defined as any member of the avian family Psittacidae (the single family of the Psittaciformes), distinguished by the short, stout, strongly hooked beak.

Recently, the hormone inhibin has been studied as a potential means for increasing ovulation in mammals. Inhibin is a peptide hormone primarily produced by the gonads, and more particularly by growing follicles. In mammals, it functions as an inhibitory feedback regulator of pituitary follicle-stimulating hormone ("FSH") secretion. While inhibin's existence was first postulated over 60 years ago, its chemical isolation was only recently achieved.

Mammalian inhibin is a dimeric protein hormone which is composed of an α-subunit (molecular weight 18,000) and a β-subunit (molecular weight 14,000). The α-subunit is unique to inhibin as dimers of the β-subunit form activin, a hormone which releases FSH from the pituitary gland. The β-subunit exists in two forms (β_{A} and β_{B}), which are distinct but quite similar. Therefore, depending on the β-subunit involved, inhibin exists as inhibin-A or inhibin-B. Both subunits α and β, when joined by disulfide bonds, are required for biological activity in suppressing follicle-stimulating hormone ("FSH") secretion from the pituitary. The amino acid sequence of the α-subunit of inhibin exhibits approximately 80-90% similarity among the porcine, bovine, human, murine, and domestic chicken species. Excellent reviews on the isolation, production, assay, and biological actions of inhibin are available in Risbridger et al., *Current Perspectives of Inhibin Biology,* Acta Endocrinologica (Copenh). 122:673-682, (1990); and Rivier, C., et al., *Studies of the Inhibin Family of Hormones: A Review,* Hormone Research, 28: 104-118 (1987).

In mammals and birds, FSH plays a role in follicular growth and development, while leuteinizing hormone ("LH") is believed to induce ovulation. Several brain and gonadal factors (peptide and steroid hormones) interact to control gonadotropin hormone release. Of these factors, gonadotropin-releasing hormone ("GnRH") and inhibin exert opposite controls on pituitary FSH secretion in mammals. Gonadotropin-releasing hormone is a brain decapeptide which acts to stimulate FSH and LH secretion, while inhibin is a gonadal protein which apparently acts to selectively inhibit FSH secretion in mammals.

A basic knowledge of the avian ovulatory process is needed to understand the role of inhibin in the endocrine control of ovulation in birds. Growing follicles on the functionally mature ovary of the domestic hen exist in a distinct size hierarchy. A typical ovary contains four to six large, two to four centimeter in diameter, yolk-filled follicles (F₁ to F₄, F₆), accompanied by a greater number of smaller, two to ten millimeter, yellow follicles, and numerous very small white follicles. The largest preovulatory follicle (F₁) is destined to ovulate the next day, the second largest (F₂) on the following day (approximately 26 hours later), and so on. The control of follicular recruitment and development within this hierarchy is poorly understood. Pituitary gonadotropin involvement has been proven, yet the role of inhibin in the control of avian gonadotropin secretion and control of ovulation remains unclear.

A recent strategy to induce hyper-ovulation in mammalian species has been the development of methods which involve neutralization of endogenous inhibin activity. For example, the active immunization of mammals against various inhibin-containing compounds has been studied. Immunoneutralization of inhibin has been associated with increased ovulation rates in heifers, sheep, gilts, and rats.

Accelerated ovulation rates found in mammals vaccinated with antigenic inhibin preparations is thought to be a consequence of elevated plasma FSH levels which leads to enhanced ovarian follicular development. A variety of antigens have been used as vaccines in studies which demonstrated an elevation in ovulation rate of mammals. Some of the antigens tested in mammals include: recombinant DNA derived fragments of the inhibin α-subunit (Wrathall et al., *Effects of active immunization against a synthetic peptide sequence of the inhibin α-subunit on plasma gonadotrophin concentrations, ovulation rate and lambing rate in ewes,* J. Reprod. Fert., 95:175-182, 1992; and Meyer et al., *Antiserum to an Inhibin Alpha-Chain Peptide Neutralizes Inhibin Bioactivity and Increases Ovulation Rate in Sheep,* Scientific Journal Series of the Minnesota Agric. Exp. Sta., paper No. 17,103, 1991), synthetic peptide replicas of the N-terminal sequence of bovine inhibin α-subunit coupled to ovalbumin (Glencross et al, *Effect of active immunization of heifers against inhibin on plasma FSH concentrations, ovarian follicular development and ovulation rate,* Journal of Endocrinology, 134, 11-18, 1992), synthetic peptide sequences of bovine inhibin α-subunit conjugated to human serum albumin (Morris, et al, *Effect of immunization against synthetic peptide sequences of bovine inhibin α-subunit on ovulation rate and twin-calving rate in heifers,* Journal of Reproduction and Fertility, 97:255-261, 1993), and partially purified inhibin from bovine follicular fluid (Morris et al, *Effect of Immunizing Prepuberal Lambs of Low and High Ovulation Rate Genotypes with Inhibin Partially Purified From Bovine Follicular Fluid,* Theriogenology, Vol. 35 No. 2, 1991).

Despite conflicting data on how levels of FSH fluctuated during the ovulatory cycle, in all cycling mammals studied, immunoneutralization of endogenous inhibin consistently enhanced ovarian follicular development and ovulation rate, regardless of the antigen used or the mammalian species challenged.

As stated above, inhibin involvement in the regulation of reproductive function in avian species remains unclear. Thus far, published reports have been restricted to the reproductive function of inhibin in domestic fowl. The bulk of this literature supports the theory that inhibin likely exerts parallel physiological roles in fowl to those documented in mammals: in hens, inhibin may serve as a regulator of follicular recruitment and/or development. However, in birds, inhibin's involvement in the control of ovulation rate may or may not be through suppression of pituitary FSH secretion. For example, although low egg producing hens have been found to have higher levels of inhibin in plasma and the granulosa cell layers of preovulatory follicles than high egg producing hens, no difference has been found in plasma FSH levels associated with the rate of egg laying. Wang et al, *Increase in Ovarian α-Inhibin Gene Expression and Plasma Immunoreactive Inhibin Level is Correlated with a Decrease in Ovulation Rate in the Domestic Hen,* General and Comparative Endocrinology, 91, 52-58, (1993). This reference, therefore, suggests that in hens the ovulation rate-related changes in inhibin α-subunit gene expression and plasma immunoreactive inhibin level do not directly affect ovulation rate through a modulation of plasma FSH level. Further, in Johnson, P. A., Inhibin *in the Hen,* Poultry Science, 72:955-958, (1993), a bovine RIA system was used to successfully assess immunoreactive inhibin in the plasma of hens, however no significant peak of immunoreactive inhibin was detected throughout the ovulatory cycle in spite of a preovulatory surge of LH. Accordingly, the role of inhibin in folliculogenesis in birds remains unclear.

Recently, the α-subunit of chicken inhibin was successfully cloned and sequenced. Wang and Johnson, *Complementary Deoxyribonucleic Acid Cloning and Sequence Analysis of the α-Subunit of Inhibin from Chicken Ovarian Granulosa Cells,* Biology of Reproduction, 49, 1-6*,* (1993). Comparison of the avian inhibin sequence to known mammalian inhibin α-subunit sequences showed an 86-89% homology. Northern blot analysis using two isolated probes (cINA₆ and cINA₁₂) revealed that the inhibin α-subunit is expressed in chicken ovarian granulosa cells but not in chicken brain, kidney, liver or spleen tissues.

Therefore, the biology of inhibin in birds remains poorly understood, and the responses of birds to challenges with antigenic inhibin has not yet been attempted or monitored. Accordingly, as the ratite market is substantially limited by the sub-optimal egg laying rates of many ostriches , emus, and rhea, what is needed is a composition and method for increasing the egg production of these birds. There is also a need for a composition and method for accelerating the onset of egg lay in avians. A need also exists for a composition and method for increasing the egg lay of a bird over its lifetime. Also, there is a continuing need for a rapid, simple, reliable, cost-effective method for determining whether a bird has immunologically responded to a challenge with an antigenic inhibin composition. Further, there remains a need for a rapid, simple, reliable, cost-effective method for determining whether a bird is hormonally pre-dispositioned to be a high-level or low-level egg producer.

### Summary of the Invention

The present invention is directed to a composition and method for making a heterologous protein comprising alpha-subunit avian inhibin, or a fragment thereof, and a carrier protein. The inhibin protein, or fragment thereof, is alpha-subunit avian inhibin. The carrier protein, includes, but is not limited to, maltose binding protein or bovine serum albumin, among others. The preferred carrier protein is maltose binding protein.

The heterologous protein can be either inhibin conjugated to the carrier protein or inhibin fused to the carrier protein. The method of producing the fused heterologous protein comprises inserting cDNA which is encoded for expressing inhibin, or a fragment thereof, into a vector which contains coding information for the production of a carrier protein. After inserting the vector into an expression system, the fused heterologous protein is expressed by the system. Preferably, the heterologous protein is comprised of ratite alpha-subunit inhibin, such as ostrich inhibin, emu inhibin, and rhea inhibin.

The present invention is also directed to a method of increasing egg production in birds via the administration of the heterologous protein of the present invention which comprises alpha-subunit avian inhibin protein, or a fragment thereof, and a carrier protein. The method comprises administering an effective amount of the protein to a bird such that egg production in the bird is increased. Preferably, an immunological response occurs in the animal directed against the protein. More preferably, the immunological response which occurs in the bird is also directed against the inhibin protein produced by the bird (endogenous inhibin). The method increases egg production in female birds which produce inhibin, such as ratites. More particularly, this method increases egg production in female ratites such as ostriches, emus, and rhea.

The present invention is also directed to a method of accelerating the onset of egg lay in a female bird, comprising the step of administering an effective amount of a heterologous protein comprising alpha-inhibin avian inhibin protein, or a fragment thereof, and a carrier protein, to a female bird such that the onset of egg lay is accelerated in the bird. The present invention is also directed to a method of increasing the lifetime total egg lay of a female bird, comprising the step of administering an effective amount of a heterologous protein comprising alpha-subunit avian inhibin protein, or a fragment thereof, and a carrier protein, to a female bird such that the lifetime total egg lay of the bird is increased.

In the context of the present invention it is provided a method of producing antibodies directed against the heterologous protein of the present invention. Generally, the method of producing antibodies directed against the heterologous protein comprises administering an effective amount of a heterologous protein comprising alpha-subunit avian inhibin protein, or a fragment thereof, and a carrier protein, to an animal such that an immunological response occurs in the animal against the heterologous protein. Next, a blood sample is obtained from the animal, and then antibodies directed against inhibin are isolated from the serum of the blood sample. Preferably, the antibody is isolated from the serum of the blood sample by passing the serum through a column containing effective amounts of the carrier protein to separate the antibody from the serum.

Further in the context of the present invention it is provided a rapid, simple, reliable, cost-effective method for determining whether an animal is hormonally pre-dispositioned to be a high-level or low-level egg producer. The method comprises determining the amount of inhibin produced by an animal, which is correlated to the egg production capability of the animal. Briefly described, the method of determining the quantity of inhibin in the blood of an animal, comprises withdrawing a blood sample from an animal and contacting the blood sample with anti-inhibin antibodies that are specifically directed against the animal's endogenous inhibin. The blood sample is contacted with the anti-inhibin antibodies under conditions which allow the antibodies to selectively interact with any inhibin that is present in the sample. Next, uninteracted antibodies are removed from interacted antibodies, and the quantity of antibodies that are interacted is determined..

In the context of the present invention it is also provided a rapid, simple, reliable, cost-effective method for determining whether an animal has immunologically responded to a challenge with an inhibin composition. Briefly described, the method comprises binding inhibin or the heterologous protein of the present invention, to a solid phase, and contacting the immobilized inhibin with a sample of blood from the animal to be tested. The sample is contacted with the immobilized inhibin under conditions wherein the inhibin will selectively interact with any anti-inhibin antibodies in the sample. After removing uninteracted antibodies from the sample, a quantity of labeled antibodies from a second animal which are directed against a class of the first animal's antibodies are added. The labeled antibodies which are directed against the animal antibodies will then selectively interact with the antibodies which are bound to the immobilized inhibin. After the removal of the uninteracted labeled antibodies, the presence or quantity of interacted labeled antibodies is determined by visualizing the label. Thus, the method detects the presence and quantity of antibodies directed against inhibin in an animal, and therefore determines if the animal has immunologically responded to the administration of a composition comprising inhibin.

Accordingly, it is an object of the present invention to provide a inhibin composition which induces an immunological response in an animal upon its administration to an animal.

It is yet another object of the present invention to provide a heterologous protein comprising alpha-subunit avian inhibin protein, or a fragment thereof, and carrier protein.

It is a further object of the present invention to provide a composition comprising a fused heterologous protein comprised of alpha-subunit avian inhibin, or a fragment thereof.

It is also an object of the present invention to provide a method for producing a heterologous protein wherein one of the proteins is comprised of alpha-subunit avian inhibin, or a fragment thereof.

It is another object of the present invention to provide a method for producing a fused heterologous protein wherein one of the proteins is comprised of alpha-subunit avian inhibin, or a fragment thereof.

It is further an object of the present invention to provide a method for producing a heterologous protein comprising alpha-subunit avian inhibin protein, or a fragment thereof, and a carrier protein.

It is an object of the present invention to provide a method for accelerating the onset of egg lay (puberty) in birds.

It is also an object of the present invention to provide a method for accelerating the onset of egg lay (puberty) in ratites such as ostriches and emus.

It is further an object of the present invention to provide a method for accelerating the onset of egg lay (puberty) in psittaciformes.

It is also an object of the present invention to provide a method for increasing the total egg lay of a bird over its lifetime

It is another object of the present invention to provide a method for increasing the egg production rate of birds.

It is a further object to provide a method for increasing the egg production rate of ratites such as ostriches and emus.

It is also an object of the present invention to provide a method for increasing the egg production rate of chickens.

These and other objects, features and advantages of the present invention will become apparent after a review of the following detailed description of the disclosed embodiments and the appended claims.

### Brief Description of the Drawings

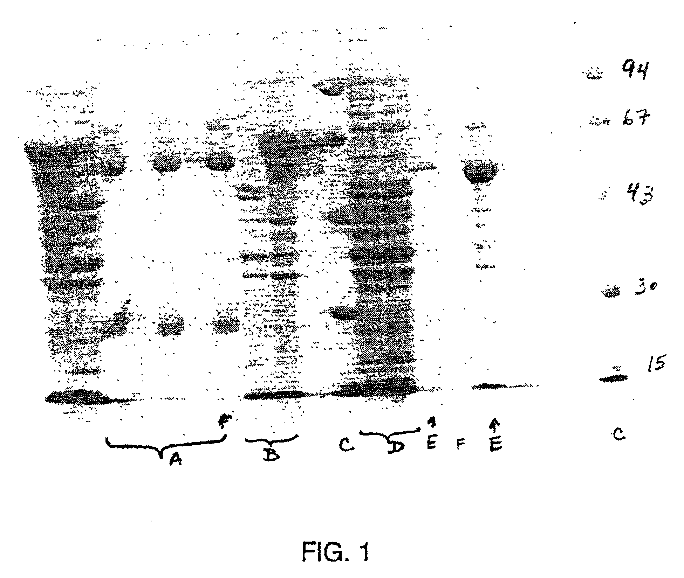

Figure 1 is an SDS-PAGE gel wherein A is ostrich anti-(chicken inhibin-maltose binding protein) antibodies, B is plasmid pMAL™-c vector standard, C is protein molecular weight standards, D is the actual pMAL™-c vector used in the preparation of the fused heterologous protein, E is the purified fused chicken inhibin-maltose binding protein (heterologous protein) of the present invention, and F is eluent from a purification that was not loaded with the heterologous protein.

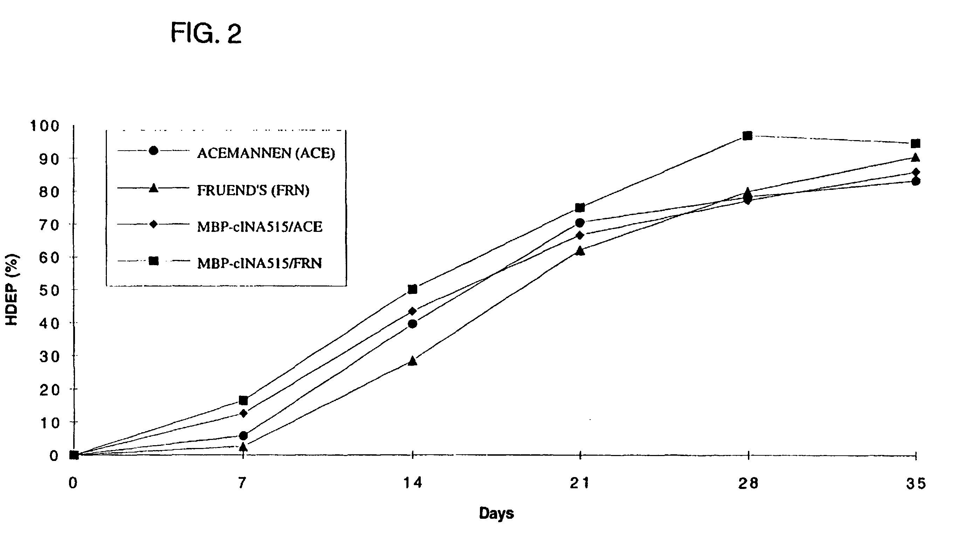

Figure 2 is an illustration of the effect of inhibin α-subunit immunoneutralization on hen-day egg production ("HDEP") in Japanese Quail using maltose binding protein fused with the protein encoded by cINA₅₁₅. More particularly, Figure 2 is a graphic representation of the data in Table 2 in Example 8.

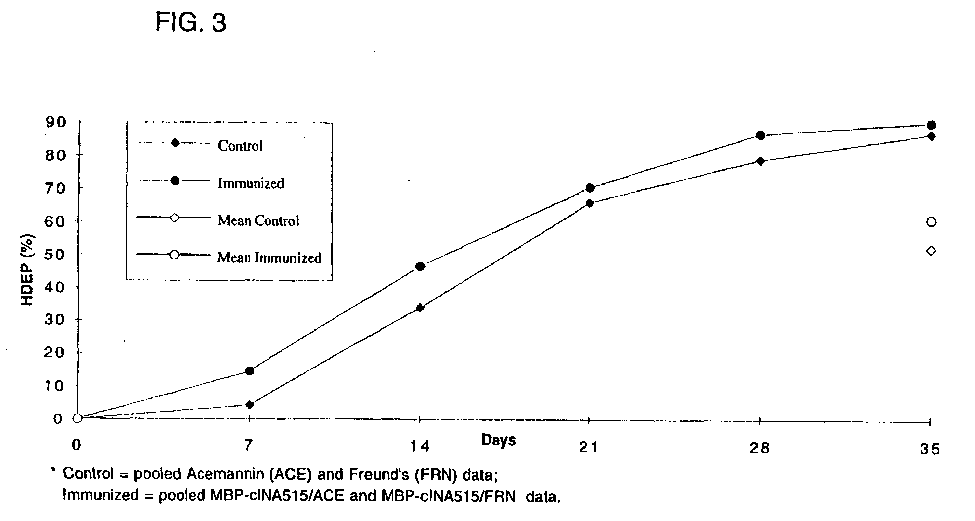

Figure 3 is an illustration of the effect of inhibin α-subunit immunoneutralization on hen-day egg production ("HDEP") in Japanese Quail using maltose binding protein fused with the protein encoded by cINA₅₁₅, when only two injection treatments were considered: control; and inhibin immunized. More particularly, Figure 3 is a graphic representation of the data in Table 3 in Example 8.

### Detailed Description

The term "bird" or "fowl," as used herein, is defined as a member of the *Aves* class of animals which are characterized as warm-blooded, egg-laying vertebrates primarily adapted for flying. The term "Ratite," as used herein, is defined as a group of flightless, mostly large, running birds comprising several orders and including the emus, ostriches, kiwis, and cassowaries. The term "Psittaciformes", as used here, include parrots, and are a monofamilial order of birds that exhibit zygodactylism and have a strong hooked bill. A "parrot" is defined as any member of the avian family Psittacidae (the single family of the Psittaciformes), distinguished by the short, stout, strongly hooked beak.

The term "egg" is defined herein as a large female sex cell enclosed in a porous, calcarous or leathery shell, produced by birds and reptiles. "Egg production by a bird or reptile", as used herein, is the act of a bird laying an egg, or "oviposition". The term "ovum" is defined as a female gamete, and is also known as an egg. Therefore, egg production in all animals other than birds and reptiles, as used herein, is defined as the production and discharge of an ovum from an ovary, or "ovulation". Accordingly, it is to be understood that the term "egg" as used herein is defined as a large female sex cell enclosed in a porous, calcarous or leathery shell, when it is produced by a bird or reptile, or it is an ovum when it is produced by all other animals.

The terms "egg lay" and "puberty", in reference to birds are used interchangeably herein, and denote when a bird lays its first egg. Accordingly, "accelerating the onset" of egg lay or puberty in avians, as used herein, denotes inducing an earlier date of first egg lay than a bird would normally have.

It is to be understood that a method of "decreasing cholesterol levels" of an egg, as used herein, denotes a method of inducing a bird to lay one or more eggs having a lower cholesterol content than the average cholesterol content of eggs laid by birds of the same species.

The phrase "lifetime total egg lay" of a bird is defined as the total number of eggs laid by a bird during its entire life span. The phrase "hen day egg production" or "HDEP", as used herein, is defined as the number of eggs laid by a particular group of hens per day.

In contrast to the term bird or fowl, the term "mammal", as used herein, is defined as a member of the class *Mammalia* which is a large class of warm-blooded vertebrates containing animals characterized by mammary glands, a body covering of hair, three oscicles in the middle ear, a muscular diaphragm separating the thoracic and abdominal cavities, red blood cells without nuclei, and embryonic development in the allantois and amnion.

A heterologous protein, as used herein, is defined as a protein comprised of alpha-subunit avian inhibin protein, or an fragment thereof, and a carrier protein. It is to be understood that the terms "inhibin" and "fragment of inhibin" are used interchangeably in the heterologous protein composition, the method of making the heterologous protein, and the method of using the heterologous protein of the present invention.

It is also to be understood that "cINA₅₁₅", as used herein, denotes a 515 base pair sequence (SEQ ID NO:1), or denotes a 516 base pair sequence (SEQ ID NO:3), or denotes the corresponding sequences of cINA6 listed on page 3 (starting at about base pair position number 790 and ending at about base pair postion number 1310), of Wang and Johnson, *Complementary Deoxyribonucleic Acid Cloning and Squence Analysis of the α-Subunit of Inhibin from Chicken Ovarian Granulosa Cells,* Biology of Reproduction, 49, p.1-6, 1993, obtained from chicken genomic DNA. cINA₅₁₅ codes for a portion of the alpha-inhibin subunit of a chicken, such as SEQ ID NO:2 or SEQ ID NO:4. As used herein, "MBP-cINA₅₁₅" is the protein conjugate that is expressed from a recombinant host cell, after cloning cINA₅₁₅ into a recombinant host cell and expressing a fused heterologous protein comprising maltose binding protein ("MBP") and the inhibin protein alpha-subunit fragment encoded by cINA₅₁₅. Accordingly, "cINA₅₁₅" denotes a nucleotide sequence, and "MBP-cINA₅₁₅" denotes a fused heterologous protein.

A fused heterologous protein, as used herein, is defined as a protein comprised of alpha-subunit avian inhibin protein, or a fragment thereof, fused to a carrier protein. The fused heterologous protein is expressed from an expression system comprising a fused gene product which contains a gene encoded for the expression of alpha-subunit avian inhibin protein, or a fragment thereof, fused to a gene encoded for expression of a carrier protein. "Fused gene product", as used herein, is defined as the product resulting from the fusion of the gene encoded for the expression of alpha-subunit avian inhibin protein, or a fragment thereof, and the gene encoded for the expression of a carrier protein.

A conjugated heterologous protein, as used herein, is defined as a protein comprised of alpha-subunit avian inhibin protein, or a fragment thereof, conjugated to a carrier protein. The conjugated heterologous protein is produced by a chemical reaction which links the alpha-subunit avian inhibin protein to the carrier protein with a covalent bond.

An immunological response of an animal to a substance which has been administered to the animal, as used herein, is defined as the cell-mediated and/or humoral response of an animal which is specifically directed against the substance.

The term "selectively interact", as used herein, is defined as where two objects associate with each other by a covalent bond, a noncovalent bond, electrostatically, a receptor-ligand interaction, a enzyme-substrate interaction, or by other binding or attachment means. The association is selective in that the two objects interact in a specific manner, in a specific position, or only with each other.

This invention relates in general to a composition used in a method of accelerating the onset of egg lay in birds. The composition is comprised of a heterologous protein comprising alpha-subunit avian inhibin protein, or a fragment thereof, and a carrier protein. The inhibin is limited to, bird inhibin. The bird inhibin can be selected from, but not limited to, ostrich inhibin, emu inhibin, rhea inhibin, cassowary inhibin, kiwi inhibin, turkey inhibin, quail inhibin, chicken inhibin, and inhibin from members of the order psittaciformes. A preferred inhibin is ratite inhibin. An especially preferred inhibin is ostrich inhibin. Another preferred inhibin is emu inhibin. Yet another preferred inhibin is rhea inhibin. Another preferred inhibin is chicken inhibin. The heterologous protein of the present invention comprises alpha-subunit inhibin protein, of a fragment thereof, and a carrier protein.

The heterologous protein of the present invention, comprising alpha-subunit avian inhibin protein, or a fragment thereof, and a carrier protein, is also used in a method of increasing the lifetime total egg lay of birds. This composition is also used in a method of increasing the rate of egg production in birds. These methods, and the method for accelerating the onset of egg lay in birds, will be discussed in more detail below.

The alpha-subunit avian inhibin, or fragment thereof, can be isolated from animal fluids, expressed from genetically engineered cells in an expression system, or synthetically produced from a series of chemical reactions. More particularly, the fragment of alpha-subunit avian inhibin can be selected from, but is not limited to the following compositions: α-subunit inhibin; recombinant DNA derived fragments of α-subunit inhibin; synthetic peptide replicas of fragments of α-subunit inhibin; synthetic peptide replicas of the N-terminal sequence of α-subunit inhibin; fragments of partially purified inhibin from follicular fluid; fragments of endogenous α-subunit inhibin; and fragments of exogenous α-subunit inhibin. As stated above, the fragment of inhibin is alpha (α)-subunit inhibin, or a fragment thereof.

The alpha-subunit avian inhibin in the heterologous protein is either fused to or conjugated with the carrier protein as described below. Where the inhibin is fused to the carrier protein, the heterologous protein is a "fused heterologous protein". Where the inhibin is conjugated to the carrier protein, the heterologous protein is a "conjugated heterologous protein". A preferred heterologous protein is a fused heterologous protein.

The identity of the carrier protein in the heterologous protein is not a critical aspect of the present invention. Any carrier protein known in the art can be used in the heterologous protein. The carrier proteins that can be used in the present invention can be selected from, but are not limited to the following group: maltose binding protein "MBP"; bovine serum albumin "BSA"; keyhole limpet hemocyanin "KLH"; ovalbumin; flagellin; serum albumin of any species; gamma globulin of any species; and polymers of D- and/or L- amino acids. A preferred carrier protein is MBP. Another preferred carrier protein is BSA if the heterologous protein will not be administered to a cow or horse. Yet another preferred carrier protein is ovalbumin if the heterologous protein will not be administered to a bird. The most preferred carrier protein is MBP. It is preferred that the carrier protein is immunogenic to the animal that it will be administered to.

The present invention also relates to a method of producing the conjugated heterologous protein of the present invention. Methods of producing conjugated proteins are well known in the art. Methods of conjugating proteins to proteins are fully described in *Antibodies, A Laboratory Manual,* edited by Ed Harlow & David Lane, Coldspring Harbor Lab (1988), which is incorporated herein by reference.

Although conjugated proteins may be used in the methods of the present invention, fusion proteins are preferred. More particularly, heterologous proteins that are fused yield a homogeneous product, wherein the different segments of the proteins are always fused in the same position, and the same amount of the segments of the proteins are fused. Also, fused heterologous proteins can be produced uniformly, inexpensively, and in large quantities. In contrast, conjugated heterologous proteins are not as uniform as fused proteins. For example, depending on what proteins are being conjugated, the conjugation reaction may yield a mixture of proteins having one or more conjugations, proteins having conjugations in different locations, or proteins that remain unconjugated. Further, some of the conjugations may render the heterologous protein sterically hindered for its intended use (*e.g*., the immunogenic portion of the protein is sterically hindered). Also, conjugation reaction conditions and reagents may degrade the proteins produced therein. For example, glutaraldehyde is commonly used in conjugation reactions, and it modifies the conformation of proteins. Further, conjugated proteins are more expensive to produce in large quantities than fused proteins.

This invention further relates to a method of producing the fused heterologous protein. The fused heterologous protein is expressed by a fusion gene product comprising a gene encoded for the expression of alpha-subunit avian inhibin protein, or a fragment thereof, and a gene encoded for the expression of a carrier protein. The fusion gene product and the method of making the fusion gene product are described more fully below. Briefly described, the method of producing the fused heterologous protein of the present invention is comprised of the steps of inserting the fusion gene product into a coding region of a plasmid, transfecting a host cell with the plasmid, and expressing the fused heterologous protein from the host cell by methods well known in the art.

Many methods of making fused heterologous proteins are known in the art. Therefore, any method known in the art can be used to produce the fused heterologous protein of the present invention. Many commercially available vector kits and expression systems can be used to prepare the fused heterologous protein of the present invention. An example of such a commercially available vector kit and expression system is pMAL™-c of New England Biolabs, Beverly Massachusetts. Cytoplasmic expression of the fused heterologous protein occurs in the pMAL™-c system. The method of producing the fused heterologous protein of the present invention from a pMAL™-c kit is fully described below in Examples 1 and 2. Other sources of vector kits and expression systems which can be used to produce the fused heterologous protein of the present invention include, but are not limited to: Pharmacia Biotech of Piscataway, New Jersey; and Clonetech, of Palo Alto, California.

The present invention further relates to a fusion gene product comprising a gene encoded for the expression of alpha-subunit avian inhibin protein, or a fragment thereof, and a gene encoded for the expression of a carrier protein. The bird inhibin gene can be selected from, but is not limited to, an ostrich inhibin gene, an emu inhibin gene, a rhea gene, a cassowary inhibin gene, a kiwi inhibin gene, a turkey inhibin gene, a quail inhibin gene, a chicken inhibin gene, an inhibin gene from any member of the order psittaciformes, an inhibin gene from any falconiformes, an inhibin gene from any piciformes, an inhibin gene from any strigiformes, an inhibin gene from any coraciformes, an inhibin gene from any ralliformes, an inhibin gene from any passeriformes, an inhibin gene from any cuculiformes, an inhibin gene from any columbiformes, an inhibin gene from any galliformes (domestic fowl), an inhibin gene from any anseriformes (geese, ducks, other water fowl), an inhibin gene from any herodiones, and an inhibin gene from any of the following birds: falcon, eagle, hawk, pigeon, parakeet, cockatoo, makaw, parrot, and perching bird (such as, song bird, jay, blackbird, finch, warbler, and sparrow).

A more preferred inhibin gene is a ratite inhibin gene. An especially preferred inhibin gene is an ostrich inhibin gene. Another preferred inhibin gene is an emu inhibin gene. Yet another preferred inhibin gene is a rhea inhibin gene. Another preferred inhibin gene is a chicken inhibin gene.

The chicken inhibin α-subunit cDNA clone (cINA6; Wang and Johnson, *Complementary deoxyribonucleic acid cloning and sequence analysis of the α-subunit of inhibin from chicken ovarian granulosa cells,* BIOLOGY OF REPRODUCTION, 49: 453-458, 1993) inserted into the EcoR 1 site of Bluescript (Stratagene, La Jolla, CA) was obtained as a gift of P. A. Johnson (Cornell University). The cINA6 clone specifically hybridized to ostrich genomic DNA in Southern assays indicating significant DNA homology between these two species (Chouljenko et al., *Expression and purification of chicken α-inhibin as a fusion protein with the E. coli maltose binding protein,* POULTRY SCIENCE, 73(Suppl. 1): 84, 1994). A DNA fragment ("cINA₅₁₅") was excised from the cINA6 clone using Pst I digestion. The cINA₅₁₅ DNA fragment encompassed most of the mature chicken inhibin α-subunit.

The ostrich inhibin α-subunit sequence was obtained by polymerase chain reaction (PCR) methods that are well known in the art. More particularly, the following primers were constructed based on the sequence reported in Wang, and were used in a PCR reaction with ostrich genomic DNA: 5'-TCTTTCGAGGAGCTGGGCTGG-3' ; and 3'-GGGCCGTGGTACGCGAGTGACGCA-5'. Approximately 75% of the DNA sequence of cINA₅₁₅ has been compared with ostrich genomic DNA. Thus far, a 100% sequence homology is evident between the chicken and ostrich DNA comparisons that have been made.

As stated above, it is to be understood that the carrier protein is not a critical aspect of the present invention. Therefore, a gene encoded to express any carrier protein can be used in the present invention. The carrier protein gene can be selected from, but is not limited to, genes encoded for expressing the following proteins: maltose binding protein "MBP"; bovine serum albumin "BSA"; keyhole limpet hemocyanin "KLH"; ovalbumin; flagellin; serum albumin of any species; gamma globulin of any species; and polymers of D- and/or L- amino acids. A preferred carrier protein gene is a gene encoded to express MBP. Another preferred carrier protein gene is a BSA gene if the resultant heterologous protein will not be administered to a cow or horse. Yet another preferred carrier protein gene is an ovalbumin gene if the resultant heterologous protein will not be administered to a bird. The most preferred carrier protein gene is a gene encoded to express MBP. The preferred carrier protein genes code for proteins that will increase both the intensity and duration of the host's immune response to the inhibin protein.

The present invention further relates to a method for making a fusion gene product comprising the step of fusing a gene encoded for the expression of alpha-subunit avian inhibin protein, or a fragment thereof, to a gene encoded for the expression of a carrier protein. Briefly described, the method of making the fusion gene of the present invention comprises the steps of isolating the desired inhibin complementary DNA (cDNA), producing double strand inhibin DNA, obtaining double strand carrier protein DNA, and fusing the double strand inhibin DNA to the double strand carrier protein DNA in a manner such that the fused DNA enables the expression of a fused heterologous protein comprising the inhibin protein, or a fragment thereof, and the carrier protein.

Many methods of isolating genes and making fusion gene products are known in the art. See, for example, Sambrook, Fritsch & Maniatis, *Molecular Cloning, A Laboratory Manual,* 2nd Ed., Cold Spring Harbor Laboratory Press, 1989, Vols. I, II, III. Therefore, any method known in the art can be used to produce the fusion gene product of the present invention. Many commercially available vector kits can be used to prepare the fusion gene product of the present invention. An example of such a commercially available vector kit is pMAL™-c of New England Biolabs, Beverly Massachusetts. The method of producing the fusion gene product of the present invention from a pMAL™-c kit is fully described below in Example 1. Other sources of vector kits which can be used to produce the fused gene product of the present invention include, but are not limited to: Pharmacia Biotech of Piscataway, New Jersey; and Clonetech, of Palo Alto, California.

As stated above, the chicken inhibin α-subunit cDNA clone (cINA6) inserted into the EcoR 1 site of Bluescript was obtained as a gift of P. A. Johnson (Cornell University). A DNA fragment ("cINA₅₁₅") was excised from the cINA6 clone using Pst I digestion. This fragment (cINA₅₁₅) was cloned in plasmid p-MALTM-c in frame with the maltose binding protein ("MBP") and a fusion protein of appropriate size (Lane E; Figure 1) was detected after IPTG (isopropyl β-D-thiogalactopyranoside) induction and SDS-PAGE. The resulting protein conjugate ("MBP-cINA₅₁₅") was used as an antigen to immunize pre-pubescent, female Japanese quail (*Coturnix coturnix japonica)* against circulating inhibin levels as is fully described in Example 8.

It has been unexpectedly discovered that the composition of the present invention accelerates the onset of egg lay in avians. This invention further relates to a method for accelerating the onset of egg lay or puberty of female avians via the administration of an effective amount of the heterologous protein of the present invention (comprising alpha-subunit avian inhibin, or a fragment thereof, and a carrier protein) such that the onset of egg lay is accelerated in the bird. The term "accelerates" denotes that egg lay of a treated bird commences at least about 3% earlier than egg lay would ordinarily commence in an untreated bird. Preferably, egg lay commences at least about 5% earlier, and more preferably commences at least about 7% earlier. Even more preferably, egg lay commences at least about 10% earlier, and most preferably commences at least about 13% earlier than egg lay would ordinarily commence in an untreated bird. It is to be understood that a "treated" bird is a bird to which the heterologous protein of the present invention has been administered. Preferably, an immunological response occurs in the bird against the inhibin. More preferably, the immunological response is also directed against the endogenous inhibin produced by the bird.

The method of the present invention can be used to accelerate the onset of egg lay in any species of female bird that produces inhibin. The female bird can be selected from, but not limited to, a ratite, a psittaciformes, a falconiformes a piciformes, a strigiformes, a passeriformes, a coraciformes, a ralliformes, a cuculiformes, a columbiformes, a galliformes (domestic fowl), an anseriformes (geese, ducks, other water fowl), and a herodiones. More particularly, the female bird can be selected from, but is not limited to, an ostrich, emu, rhea, kiwi, cassowary, turkey, quail, chicken, falcon, eagle, hawk, pigeon, parakeet, cockatoo, makaw, parrot, perching bird (such as, song bird, jay, blackbird, finch, warbler, sparrow), and any member of the order psittaciformes. A preferred bird is a ratite. A more preferred bird is an ostrich. Another preferred ratite is an emu. Yet another preferred ratite is a rhea. Another preferred bird is any member of the order psittaciformes. Yet another preferred bird is a chicken. Still another preferred bird is a quail. The method of the present invention can also be used to accelerate the onset of egg lay in species of birds that are endangered. Such endangered birds include, but are not limited to, eagles, hawks, condors, and owls.

The alpha-subunit avian inhibin and the carrier protein in the heterologous protein composition of the present invention vary according to what species of bird the composition will be administered to. It is preferred that alpha-subunit avian inhibin and maltose binding protein is used when the composition is to be administered to a bird. A preferred alpha-subunit inhibin is domestic chicken or ratite inhibin when the composition is to be administered to a ratite. More preferably, the preferred inhibin is domestic chicken or ostrich inhibin when the composition is to be administered to an ostrich.

It is to be understood that the alpha-subunit avian inhibin in the heterologous protein need not be from the same species to which the heterologous protein will be administered. For example, a heterologous protein that is administered to an ostrich can be comprised of chicken alpha-subunit inhibin and a carrier protein. It is also to be understood that the composition can further comprise adjuvants, preservatives, diluents, emulsifiers, stabilizers, and other known components that are known and used in vaccines of the prior art. Any adjuvant system known in the art can be used in the composition of the present invention. A preferred adjuvant system is Freund's incomplete adjuvant. Another preferred adjuvant system is Freund's complete adjuvant. Yet another preferred adjuvant system is a polydispersed β-(1,4) linked acetylated mannan ("Acemannan").

The heterologous protein composition of the present invention can be administered to a female bird by any means known in the art. For example, the composition can be administered subcutaneously, intraperitonealy, or intramuscularly. Preferably, the composition is injected subcutaneously. The composition can be administered to the bird in one or more doses. Preferably, the composition is administered to the bird in multiple doses wherein an initial immunization is followed by booster immunizations.

The composition of the present invention is to be administered to a female bird before the bird reaches egg lay or puberty. The preferred age at which the composition of the present invention is first administered to an animal depends upon the species of the animal involved, the mating season (if any) of an animal, upon the size of the bird, and upon the identity of the components (inhibin and carrier protein) in the composition.

The amount administered to a female bird of the heterologous protein of the present invention varies according to the species of the bird, the age and weight of the bird, when the protein is administered in relation to the breeding season (if the bird has a breeding season), and how many times the protein is to be administered. Also, the commencement of the administration schedule, or treatment schedule, varies according to the species of the bird, the average age of onset of egg lay of that species of the bird, the family history of the bird (with respect to the family's history of start of egg lay), the time of year the bird was hatched, the nutritional plane of the bird (highly fed birds come into puberty before those that are undernourished), the general health of the bird at that time of commencement, the long term health history of the bird, the presence of extreme weather conditions (prolonged excessive inclement weather such as rain, heat, or windiness that the bird is not accustomed to), housing conditions (overcrowding), and a lack of exercise.

One of ordinary skill in the art in view of the teachings of the present invention would be able to determine by routine testing the amount of heterologous protein that will be necessary to elicit an immunological response to the protein by the bird.

For example, the following is a brief summary of the method of the present invention for accelerating the onset of puberty in Japanese Quail as is fully discussed in Example 8. The average age at puberty for an untreated quail is approximately six to eight weeks. The following would be the treatment schedule for Japanese quail having an approximate body weight range of 0.1 to 0.25 pounds: primary (first) injection of 0.75 mg of the heterologous protein of the present invention on its 25th day of age; and boosters of 0.375 mg on the 32nd, 39th, 46th, 53rd, 60th, and 90th day of age.

More particularly, at 25 days-of-age, 100 quail were randomly and equally assigned to one of four injection groups (25 birds per group) as follows: (1) MBP-cINA₅₁₅ in a polydispersed β-(1,4) linked acetylated mannan ("Acemannan"), an immunostimulant vehicle, ("MBP-cINA₅₁₅/ACE"), (2) MBP-cINA₅₁₅ in Freund's adjuvant ("MBP-cINA₅₁₅/FRN"), (3) Acemannan (immunostimulant control; "ACE"), or (4) Freund's (adjuvant control; "FRN"). Birds immunized against inhibin (Groups 1 and 2) were given approximately 0.75 mg MBP-cINA₅₁₅ per bird in the appropriate control vehicle. Equivalent vehicular injection volumes (0.2 mL) of ACE or FRN were used in Groups 1 and 3 and Groups 2 and 4, respectively. All injections were given subcutaneouly using tuberculin syringes fitted with 25 ga needles. Weekly booster inhibin immunizations of approximately 0.375 mg MBP-cINA₅₁₅ per bird, or appropriate control challenges, were subsequently administered for five consecutive weeks

Beginning at 41 days-of age (considered Day 1 of the egg lay cycle), daily hen-day egg production ("HDEP") and mortality ("MORT") measures were recorded for 12 consecutive weeks. In addition, average age at first egg lay ("FIRST") and age at which hens reached 50% egg production ("FIFTY") were calculated for each of the four treatment groups. As is more fully discussed in Example 8, HDEP data were subjected to two different analyses of variance.

To date, information is preliminary as only 35 days of data have been collected. Cumulative percent HDEP rates for weekly intervals of egg lay (days 0, 7, 14, 21, and 35) during the first 35 days of study are plotted in Figure 2 (wherein the four injection groups were considered as separate treatments) and Figure 3 (wherein only non-immunized (control) versus inhibin-immunized group comparisons were made).

Mean HDEP rates during Week 1, Week 2, Week 3, Week 4, Week 5 and Weeks 1 through 5 combined for the four injection groups considered as independent treatments are given in Table 2. More particularly, the values in Table 2 are represent the percent egg lay rate. For example, in Week 1 of the study, only 5.8% of the birds in the ACE group laid an egg a day. In Week 2 of the study, 39.56% of the birds in the ACE group laid an egg a day. (The 39.56% of Week 2 includes the 5.8% from Week 1.)

Also, Table 2 shows that in Week 4, the MBP-cINA₅₁₅/FRN injection group peaked at an egg lay rate of 96.57%. Therefore, from the values in Table 2, the other three injection groups did not peak in Week four as their percent lay rate increased from Week 4 to Week 5. The column under Weeks 1-5 represents the average percent lay rate of each of the injection groups for Weeks 1 through 5.

Table 3 gives mean HDEP rates in percent during the same time frames as Table 2, wherein data is pooled for direct control versus immunized treatment comparisons. The column under Weeks 1-5 represents the average percent lay rate of the combined control groups versus the combined inhibin immunized groups for Weeks 1 through 5.

Table 4 contains FIRST egg-lay data for both of the statistical scenarios considered (*ie.,* the four injection treatment groups and the pooled data comparisons). The age of first egg lay in Table 4 represents the statistical mean of the first day of egg lay for all of the birds in each group. Note that Table 5 shows more than a five day acceleration of onset of egg lay between the birds in the FRN group and the birds in the MBP-cINA₅₁₅/FRN group. Also Table 5 shows more than a 3 day acceleration of onset of egg lay between birds in the Control group and the birds in the Immunized group. (See Example 8 for Tables 2, 3, and 4.)

An initial positive effect of inhibin immunoneutralization on production performance was extant. This effect is particularly evident in the pooled HDEP (Table 3) and FIRST (Table 4) data sets where increased numbers of observations comprising means augmented power of the statistical tests applied.

Most timed biological responses to treatments are studied for effects on onset, magnitude, and duration of response. Herein, the data are preliminary (ie., information on an entire laying cycle (approximately 12 weeks post-onset of egg lay) has yet to be collected. Therefore, a complete understanding of the underlying mechanism(s) as to how production performance is altered in quail immunized against inhibin awaits determination. However, the present data does show the effects of inhibin immunoneutralization on the initial portion of an egg lay cycle (*ie.,* onset of puberty).

The data shows that onset of puberty was accelerated in the inhibin treatment groups. This was evidenced in both the HDEP and FIRST data collected. There was a high degree of statistical confidence (P<.0095) associated with the higher mean HDEP rate found in inhibin-treated quail during Week 1 of the study, a difference that was less evident (P<.0888) during Week 2 (Table 3). This marginal treatment group difference observed during Week 2 may represent that egg lay, although delayed, was beginning to intensify in control-injected quail (See Figure 2). Consequently, the strength of the statistical difference found between the two treatment groups (inhibin versus no-inhibin) during Week 1 would expectedly become diluted with advancing time of lay. Indeed, when data from Weeks 1 through 5 were combined, a significantly higher mean HDEP rate of intermediate statistical relevance (P<.0763) was found in inhibin-treated hens.

Japanese quail have been selected for intensity of egg lay, and egg laying potential is considered to be even greater in Coturnix than in chicken hens (Single Comb White Leghorns) commercially reared for the single purpose of production of table eggs. Secondly, the mean age at which immunized quail (51.98 days-of-age) laid their first egg (FIRST) was advanced (P<.0373) by 3+ days when compared to non-immunized quail (*55*.33 days-of-age) (Table 4). This effect of inhibin immunization on acceleration of egg lay was particularly noteworthy in MBP-cINA₅₁₅/FRN-treated quail which had an average mean FIRST of 50.38 days-of-age (Table 4). These results also indicate that higher antibody titers to heterologous protein challenge (and therefore greater inhibin immunoneutralization) resulted when Freund's adjuvant was used as the vehicle for MBP-cINA₅₁₅ than when Acemannan was used.

Additional evidence supports the conclusion that inhibin immunoneutralization accelerated the onset of puberty in quail. Specifically, all (100%) of the inhibin-treated hens have thus far (*ie.,* by 76 days-of-age or Day 35 of the experiment) instigated egg lay, while two of the control-treated hens have yet to commence egg lay. This suggests that if the two remaining hens never initiate egg lay, then inhibin immunoneutralization may have induced lay in hens that would have otherwise never laid. Furthermore, if these same two birds never lay, or even if the remaining two hens begin egg lay sometime before the end of the study (by 124 days-of-age), then the 3+ day difference in the FIRST egg lay data reported herein will, by mathematical necessity, further widen. In addition, while statistical testing of FIFTY data is as of yet contraindicated (because approximately 23 % of the hens have yet to reach 50% HDEP), the majority of these late-layers (approximately 57 %) are members of the two control (non-immunized) groups. This finding further supports the HDEP and FIRST data that suggest onset of puberty was accelerated in inhibin-immunoneutralized quail hens.

Accordingly, the above data shows that the inhibin composition of the present invention accelerates the onset of puberty in Japanese Quail. Since Japanese Quail are an acceptable animal model for chickens with respect to their reproductive systems, the above data indicates that the method of the present invention will also accelerate the onset of egg lay in chickens. Accordingly, the method of the present invention will result in an egg producer being able to produce more eggs with lower feed costs.

The following is a brief summary of the method of the present invention for accelerating the onset of puberty in ostriches as is discussed in Example 9. The average age at puberty for untreated ostriches is between approximately 28 and 32 months. The following would be the treatment schedule for ostriches having an approximate body weight range of 150 to 300 pounds: primary (first) injection of 5.0 mg of the heterologous protein of the present invention on its 26th month of age; and boosters of 2.5 mg on the 27th, 28th, 30th, 32nd, 34th, and 36th month of age.

The following is a brief summary of the method of the present invention for accelerating the onset of puberty in emu as is discussed in Example 10. The average age at puberty for untreated emu is approximately 20 months. The following would be the treatment schedule for emu having an approximate body weight range of 50 to 90 pounds: primary (first) injection of 3.0 mg of the heterologous protein of the present invention on its 18th month of age; and boosters of 1.5 mg on the 19th, 20th, 22nd, 24th, 26th, and 30th month of age.

The following is a brief summary of the method of the present invention for accelerating the onset of puberty in chickens as is discussed in Example 11. The average age at puberty for an untreated chicken is approximately 20 weeks. The following would be the treatment schedule for a chicken having an approximate body weight range of 2.0 to 3.5 pounds: primary (first) injection of 1.5 mg of the heterologous protein of the present invention on its 15th week of age; and boosters of 0.75 mg on the 17th, 20th, 24th, 30th, 40th, and 50th week of age.

The following is a brief summary of the method of the present invention for accelerating the onset of puberty in turkeys as is discussed in Example 12. The average age at puberty for an untreated turkey is approximately 30 weeks. The following would be the treatment schedule for a turkey having an approximate body weight range of 9.0 to 12 pounds: primary (first) injection of 2.0 mg of the heterologous protein of the present invention on its 28th week of age; and boosters of 1.0 mg on the 29th, 30th, 34th, 38th, 46th, and 54th week of age.

The following is a brief summary of the method of the present invention for accelerating the onset of puberty in parrots as is discussed in Example 13. The average age at puberty for an untreated parrot is approximately 30 months. The following would be the treatment schedule for a parrot having an approximate body weight range of 0.5 to 1.25 pounds: primary (first) injection of 0.75 mg of the heterologous protein of the present invention on its 28th month of age; and boosters of 0.375 mg on the 29th, 30th, 32nd, 34th, 36th, and 38th month of age.

Another embodiment of the present invention relates to a method for increasing egg production in animals via the administration of an effective amount of the heterologous protein of the present invention (comprising alpha-subunit avian inhibin, or a fragment thereof, and a carrier protein) such that egg production is increased in the bird. The term "increases" denotes that egg production of a treated bird increases at least about 3% with respect to the amount of egg production in an untreated bird. Preferably, egg production increases at least about 7%, and more preferably increases at least about 12%. It is to be understood that a "treated" bird is a bird to which the heterologous protein of the present invention has been administered. Preferably, an immunological response occurs in the animal against the inhibin. Preferably, the immunological response is also directed against the endogenous inhibin produced by the animal.

The method of the present invention may be used to increase the egg production of a bird. The female bird can be selected from, but not limited to, a ratite, a psittaciformes, a falconiformes a piciformes, a strigiformes, a passeriformes, a coraciformes, a ralliformes, a cuculiformes, a columbiformes, a galliformes (domestic fowl), an anseriformes (geese, ducks, other water fowl), and a herodiones. More particularly, the female bird can be selected from, but is not limited to, an ostrich, emu, rhea, kiwi, cassowary, turkey, quail, chicken, falcon, eagle, hawk, pigeon, parakeet, cockatoo, makaw, parrot, perching bird (such as, song bird, jay, blackbird, finch, warbler, sparrow), and any member of the order psittaciformes. A preferred bird is a ratite. A more preferred bird is an ostrich. Another preferred ratite is an emu. Yet another preferred ratite is a rhea. Another preferred bird is any member of the order psittaciformes. Yet another preferred bird is a chicken. Still another preferred bird is a quail. The method of the present invention can also be used to increase egg lay in species of birds that are endangered. Such endangered birds include, but are not limited to, eagles, hawks, condors, and owls.

The alpha-subunit avian inhibin and the carrier protein in the heterologous protein composition of the present invention varies according to what species of animal the composition will be administered to. The heterologous protein of the present invention has been fully discussed above. It is preferred that alpha-subunit avian inhibin and maltose binding protein is used when the composition is to be administered to a bird. A preferred inhibin is domestic chicken or ratite inhibin when the composition is to be administered to a ratite. More preferably, the preferred inhibin is domestic chicken or ostrich inhibin when the composition is to be administered to an ostrich.

It is to be understood that the alpha-subunit avian inhibin in the heterologous protein need not be from the same species to which the heterologous protein will be administered. For example, a heterologous protein that is administered to an ostrich can be comprised of chicken inhibin and a carrier protein. It is also to be understood that the composition can further comprise adjuvants, preservatives, diluents, emulsifiers, stabilizers, and other known components that are known and used in vaccines of the prior art. Any adjuvant system known in the art can be used in the composition of the present invention. A preferred adjuvant system is Freund's incomplete adjuvant. Another preferred adjuvant system is Freund's complete adjuvant.

The heterologous protein composition of the present invention can be administered to a bird by any means known in the art. For example, the composition can be administered subcutaneously, intraperitonealy, or intramuscularly. Preferably, the composition is injected subcutaneously. The composition can be administered to the animal in one or more doses. Preferably, the composition is administered to the animal in multiple doses wherein an initial immunization is followed by booster immunizations.

The composition can be administered to a female bird at any time before the animal ceases to ovulate due to disease or age. The preferred age at which the composition of the present invention is administered to a bird depends upon the species of the animal involved, the mating season (if any) of an animal, and upon the purpose of the administration of the composition.

For example, where the composition is administered to increase egg production of agricultural animals which have breeding seasons, the preferred time of administering the composition is prior to the start of the breeding season. In contrast, where the composition is to be administered to a mature bird which has a suppressed egg production rate, then the composition would be administered at the time that the suppression is recognized as problematic.

For example, although the heterologous protein can be administered to a bird such as a ratite at any age, immunizing the bird during the six months prior to the bird's first breeding season is preferable. It is understood by those of ordinary skill in the art that average female birds initiate egg lay during the first breeding season. It is even more preferable to immunize the bird approximately six months prior to the bird's first breeding season, and then to administer booster immunizations at one month intervals prior to the bird's first breeding season. It is most preferable, to immunize the bird approximately six months prior to the bird's first breeding season, and then to administer booster immunizations at one month intervals for six months.

The amount administered to a bird of the heterologous protein of the present invention varies according to the species of the animal, the age and weight of the animal, when the protein is administered in relation to the breeding season (if the animal has a breeding season), and how many times the protein is to be administered. One of ordinary skill in the art in view of the teachings of the present invention would be able to determine by routine testing the amount of heterologous protein that will be necessary to elicit an immunological response to the protein by the animal.

For example, for best results in increasing the egg production of a female ostrich, a primary immunization is administered to the ostrich approximately 6 months before its first breeding season, and then booster immunizations are administered at one month intervals for six months. The primary immunization comprises between approximately 0.5 to 4.5 mg of the heterologous protein of the present invention. The booster immunizations comprise between approximately 0.30 to 3.0 mg of the heterologous protein of the present invention. Preferably, the primary immunization comprises between approximately 1.5 to 3.0 mg of the heterologous protein of the present invention. The booster immunizations comprise between approximately 0.75 to 1.5 mg of the heterologous protein of the present invention. It is also preferable that the heterologous protein is emulsified in Freund's Complete Adjuvant (Sigma Chemical Co., St. Louis, MO) in the primary immunization, and that the heterologous protein is emulsified in Freud's Incomplete Adjuvant (Sigma) in the booster immunizations. Even more preferably, the heterologous protein composition is injected subcutaneously. Most preferably, the heterologous protein composition is injected subcutaneously at three sites along the upper thigh region of the ostrich.

The method of the present invention of increasing egg production in birds provides a greater increase in egg production in species that have not been genetically selected for the trait of prolific egg laying. For example, chickens have been genetically selected for maximum egg production since the late 1920s. See, for example, Jull, M.A, 1932, *Poultry Breeding,* John Wiley & Sons. In terms of the short life span of a chicken, a great deal of selection for this trait occurred over the period of time from approximately 1928 to the present. In contrast, ratites and psittaciformes, and most other exotic birds, have not been genetically selected for the trait of prolific egg laying. Also, birds that are endangered have also not been genetically selected for the trait of prolific egg laying. Accordingly, as chickens are already genetically excellent egg layers, the amount of improvement that can be seen with the method of the present invention is limited in comparison to birds that are genetically poor to medium egg layers. Therefore, a much greater amount of improvement in the rate of egg laying is seen with the method of the present invention with birds that have not been genetically selected for prolific egg laying, such as, ratites, psittaformes, other exotic birds, and endangered birds.

The immunization of a bird with the heterologous protein of the present invention induces the animal to produce antibodies selectively directed against the heterologous protein. Preferably, the immunization also induces the animal to produce antibodies selectively directed against endogenous inhibin. The production of such antibodies by a bird reduces the time to the onset of puberty or egg lay. The production of such antibodies by the animal also enhances the animal's egg production capability as the antibodies neutralize the biological activity of inhibin in the animal's blood stream.

Not wanting to be bound by the following theory, it is believed that the α-subunit of inhibin binds to FSH receptors and therefore competitively inhibits the binding of FSH with such receptor sites. Reducing the levels of inhibin that may bind with receptor sites, therefore increases the biological effect of the FSH in the animal as there is reduced competition for the FSH receptor sites. It is thought that the antibodies neutralize the inhibin by interacting with the circulating inhibin, thereby stearically interfering with the binding of the interacted inhibin to the FSH receptor sites.

Surprisingly, the composition of the present invention can also be used to increase the lifetime total egg lay of birds. The term "increase" denotes that the total lifetime egg lay of a treated bird increases at least about 3% with respect to the total lifetime egg lay of an untreated bird. Preferably, total lifetime egg lay increases at least about 7%, and more preferably increases at least about 12%. Most preferably, total lifetime egg lay increases at least about 15%. It is to be understood that a "treated" bird is a bird to which the heterologous protein of the present invention has been administered. Briefly described, the lifetime total egg lay of birds would be increased by administering an effective amount of the heterologous protein of the present invention to induce an immunological response thereto, and thereafter administering an effective amount of the heterologous protein (boosters) to maintain maximum, or higher than normal, egg lay. More particularly, if the heterologous protein of the present invention is continually administered to the female bird, in the approximate dosages as disclosed in the methods described above for accelerating the onset of egglay or puberty, the lifetime total egg lay of the bird will be increased in comparison to if the bird was not treated with the composition of the present invention.

Generally, the method of the present invention results in a greater increase in lifetime total egg lay in species of birds that have not been genetically selected for the trait of prolific egg laying. The lifetime total egg lay of a bird is limited by the number of gametes that the bird is born with. However, some species of birds have been genetically selected for maximum ovulation or release of eggs. As stated above, for example, chickens have been genetically selected for maximum egg production since the late 1920s. In terms of the short life span of a chicken, a great deal of selection for this trait occurred over the period of time from approximately 1928 to the present. In contrast, ratites and psittaciformes, and most other exotic birds, have not been genetically selected for the trait of prolific egg laying. Also, birds that are endangered have also not been genetically selected for the trait of prolific egg laying. Accordingly, as chickens are already genetically excellent egg layers, the amount of improvement that can be seen with the method of the present invention is limited in comparison to birds that are genetically poor to medium egg layers. Therefore, a much greater increase in the lifetime total egg lay is seen with the method of the present invention with birds that have not been genetically selected for prolific egg laying, such as, ratites, psittaformes, other exotic birds, and endangered birds. The increase in lifetime total egg lay, therefore, results in the production of more eggs from the same number of birds, yielding a higher profit margin for the producer.

Unexpectedly, the composition of the present invention can also be used to decrease or eliminate the need to molt a female bird. More particularly, if the composition described above is continually administered to the female bird, as disclosed in the method above, the rate of egg lay of the bird, in comparison to if the bird was not treated with the composition of the present invention, would remain high enough so that the bird would not need to be molted to improve its rate of egg lay. It is a common practice in the art to molt a female bird, such as chicken hens (single comb white leghorns, table-grade egg producers), when its egg lay production declines such that the economic cost of maintaining the bird outweighs the economic benefit yielded by the eggs produced. To "molt" a chicken hen, the bird undergoes a period of fasting of approximately four to fourteen days until it beings to molt, *e.g.,* lose its feathers. During the molting period, the bird stops laying eggs. After the bird is placed back onto normal levels of feed, egg production recommences after a period of time. The entire molting period is approximately two months from the beginning of the fast period to the onset of the next egg-lay cycle. In effect, the egg production rate of the bird is rejuvenated. However, after molting a chicken, its rate of egg-lay in the next cycle does not equal the egg production during the first (pre-molt) egg-lay cycle. M. North and D. Bell, *Commercial Chicken Production Manual,* fourth edition, Chapter 19, Published by Van Norstrand Reinhold of New York.

For example, chickens reach egg lay at approximately 20 weeks, and produce an economically significant number of eggs for approximately 40 to 50 weeks. At the peak of egg lay, chickens produce eight to nine eggs every ten days. However, after approximately 50 weeks of egg lay, the rate of egg production decreases to approximately 60% of peak egg lay. At this point, the cost of the feed for the chicken is greater than the value of the eggs its produces. It is common practice to molt the chicken at this point, so that when the chicken recommences egg lay, its rate of egg lay is increased.

Therefore, the composition of the present invention, as it maintains the rate of egg lay at a higher level than if the bird were not treated with the composition, reduces or eliminates the need to molt a bird. The reduction or elimination of the need to molt a bird results in significant savings. More particularly, overtime a bird is molted the bird has been unproductive with respect to its feed cost before it is molted, and then it is unproductive for a period of time after feeding recommences. Maintaining the rate of egg lay at an enhanced level therefore eliminates or reduces these unproductive phases of the bird, thereby reducing the producer's costs and increasing the producer's profits. Maintaining the rate of egg lay at an enhanced level further enhances egg producer's profits as the rate of egg-lay after molting does not equal the rate of egg-lay in the first cycle of egg lay as discussed above.

Briefly described, the rate of egg lay of birds would be enhanced, thereby avoiding the need to molt the bird, by administering an effective amount of the heterologous protein of the present invention to induce an immunological response thereto, and thereafter administering an effective amount of the heterologous protein (boosters) to maintain a higher than normal rate of egg lay.

Another unexpected and surprising aspect of the present invention is that the composition of the present invention also produces more eggs having a decreased cholesterol content in comparison to the eggs laid by untreated birds. More particularly, if the composition described above is administered to a female bird, as disclosed in the method above, the cholesterol content of the eggs laid by the bird will be reduced for a longer period of time in comparison to if the bird was not treated with the composition of the present invention. Therefore, the composition of the present invention increases or poduces a greater number of low cholesterol eggs.

The terms "increase" or "greater number of' denotes that the number of low cholesterol eggs produced by a treated bird increases at least about 5% with respect to the number of low cholesterol eggs produced by an untreated bird. Preferably, the number of low cholesterol eggs produced increases at least about 10%, and more preferably increases at least about 15%. It is to be understood that a "treated" bird is a bird to which the heterologous protein of the present invention has been administered. The term "lower cholesterol" or "low cholesterol" denotes that the cholesterol content of an egg is lower than the average cholesterol content of eggs produced by that species of bird during the lifetime of such a bird by at least about 10%. Preferably, the cholesterol content of a low cholesterol egg is lower than the average by at least about 20%. More preferably, the cholesterol content of a low cholesterol egg is lower than the average by at least about 30%.

It is known that in chickens, the first five to six eggs that are laid by a female bird (hen) after it reaches puberty are lower in cholesterol than the eggs it later produces. The composition of the present invention induces the female bird to lay eggs having a lower cholesterol content for a longer period of time. Due to the health consequences linked to high cholesterol levels in the blood, there remains a need for low cholesterol egg products. Accordingly, the composition of the present invention provides the producer of eggs with a greater number of a highly sought after type of egg.

Therefore the methods of the present invention for increasing egg production and for accelerating the onset of egg lay in birds will greatly accelerate the growth of the population and therefore the market for ratites such as ostrich and emu as their suboptimal egg laying rates will be increased by the present method. The method of the present invention will also satisfy the continuing need for poultry such as domestic chickens, and their eggs.

In the context of the present invention it is provided a method of producing antibodies directed against the heterologous protein of the present invention. Generally, the method of producing antibodies directed against the heterologous protein comprises the steps of: administering an effective amount of a heterologous protein comprising inhibin protein, or a fragment thereof, and a carrier protein, to an animal such that an immunological response occurs in the animal against the heterologous protein; removing a blood sample from the animal; and then isolating any antibodies directed against inhibin from the serum of the blood sample. Preferably, the antibody is isolated from the serum of the blood sample by passing the serum through a column containing effective amounts of the carrier protein to separate the antibody from the serum. The techniques used to produce and purify antibodies directed against the heterologous protein of the present invention are well known to one of ordinary skill in the art.

It is also to be understood that the heterologous protein of the present invention can be administered to any animal dependent upon the type of antibodies that are desired. Further, it is to be understood that the inhibin can be exogenous or endogenous. Therefore, the type of inhibin in the heterologous protein of the present invention, and the species of animal the composition is administered to is determined by what type of antibody is desired. For example, heterologous protein comprising chicken alpha-subunit inhibin and maltose binding protein can be administered to an ostrich to produce ostrich anti-chicken inhibin antibodies. Also, heterologous protein comprising ostrich alpha-subunit inhibin and maltose binding protein can be administered to an ostrich to produce ostrich anti-ostrich inhibin antibodies.

In the context of the present invention it is also provided a method of determining the amount of inhibin produced by an animal, which therefore allows the determination of the egg production capability of the animal. Briefly described, the method of determining the quantity of inhibin in the blood of an animal, comprises the steps of: withdrawing a blood sample from an animal; contacting the blood sample with anti-inhibin antibodies that are specifically directed against the animal's endogenous inhibin under conditions which allow the antibodies to selectively interact to any inhibin if present in the sample; removing any uninteracted antibodies from any interacted antibodies; and determining the quantity of antibodies that are interacted.

One of ordinary skill in the art will understand that the immunoassay techniques that can be used in the above method are well known in the art. Therefore, any immunoassay technique, label, and visualization method known in the art can be used in the above method. A preferred immunoassay is ELISA ("enzyme linked immunosorbent assay"), and a preferred label is horseradish peroxidase. Another preferred label is a colored latex bead. The colored latex bead can be any color desired for visualization purposes. Preferably, the latex bead is yellow, red, blue, or green. The colored latex bead can be hollow or solid, but it preferably is hollow to minimize its weight. The size of the latex bead varies according to its intended use in immunoassays. One of ordinary skill in the art would be able to ascertain by routine testing the largest bead size that is visible yet does not interfere stearically with the immunoassay reactions. Preferably, the latex bead is less than 0.5µ in diameter, and most preferably it is less than 0.2µ in diameter.

For example, circulating inhibin concentrations in the blood of a bird can be determined using standard sandwich ELISA techniques. First, bind anti-inhibin antibodies which are directed against a portion of inhibin, or a fragment thereof, to the wells of a microtiter plate. After washing and blocking the plate, then add a quantity of blood plasma that was obtained from the bird to be tested. After allowing any inhibin in the sample, if present, to selectively interact with the immobilized anti-inhibin antibody, the sample is washed from the well of the plate. Next, add labeled anti-inhibin antibodies to the well than that are directed against a different portion of the inhibin, or a fragment thereof, than the antibody immobilized in the well. The antibody can be labeled with any label known in the art, such as horseradish peroxidase. After allowing the labeled anti-inhibin antibody to selectively interact with any immobilized inhibin, any uninteracted labeled anti-inhibin antibodies are removed by washing. The amount of inhibin present in the plasma sample is determined by using the appropriate visualization means for the label used in the ELISA to quantify the amount of immobilized labeled anti-inhibin antibody in the well. Standard positive and negative controls are to be run simultaneously in neighboring plate wells.

It is to be understood that the reproductive potential of any animal which produces inhibin can be determined by the above method. This method can be used to determine the amount of inhibin produced by a female animal of any species that produces inhibin. The animal can be a bird, mammal, or reptile, among others. More specifically, the mammal can be selected from the group consisting of, but not limited to, a cow, human, horse, cat, dog, sheep, mink, fox , otter, ferret, raccoon, and pig. The bird can be selected from, but is not limited to, an ostrich, emu, and chicken. A preferred animal is a bird. A more preferred animal is a ratite. An especially preferred animal is an ostrich. Another preferred animal is an emu. Yet another preferred animal is a chicken.

Animals having high levels of inhibin have low reproductive potential, and depending upon the species involved and whether they are raised for agricultural purposes, such a low egg producing animal may be taken to slaughter instead of being kept for breeding purposes. In contrast, those agriculturally raised animals having lower amounts of inhibin are higher egg producers and generally are used for breeding purposes, and are not taken to slaughter.

The inhibin level in an animal varies according to its age, species, and the time of year relative to the breeding season (if any). Therefore, the determination of the egg laying potential of an animal is relative to the these factors, and a measure of an animal's inhibin levels are most valuable when compared to the average inhibin levels of the same species of animal, of approximately the same age, at the same time of year if the animal has a breeding season.

As the amount of inhibin produced by a bird varies according to the above factors, relative amounts of inhibin are established below for different age categories of birds. Table 1 illustrates the variance of ratites such as emus and ostriches in their inhibin production dependent upon whether they are poor or good egg producers, and dependent upon the age of the ratite.

**Table 1**

| Age (Months) | Inhibin Level | Reproductive Potential |
|---|---|---|
| 6-12 | >5 | Poor |
| 6-12 | 2 - 5 | Moderate |
| 6-12 | 0 - 1.5 | Good |
| | | |
| 24+ | >7 | Poor |
| 24+ | 3 - 7 | Moderate |
| 24+ | 0 - 2.5 | Good |

In the above table, the inhibin level is relative to a standard pool value of 1 for female ratites averaging 50 to 60 eggs per breeding season, which is good, and a value of 7 for a pool of functionally non-producing female ratites which produce less than 5 eggs per season, which is poor.

In the context of the present invention it is also provides a method of producing animal antibodies directed against a class of another animal's antibodies, such as IgG. Briefly described, the method of producing antibodies in an animal which are directed against another animal's IgG, comprises the steps of: administering an effective amount of a class of a first animal's antibodies, such as those produced by the above described method, to a second animal such that an immunological response occurs in the second animal against the first animal's antibodies; withdrawing a blood sample from the second animal; and isolating the second animal's antibodies from the serum of the blood sample as described above. Preferably, the second animal is a different species than the first animal.

In the context of the present invention it is also provided a method of determining if an animal has immunologically responded to the administration of a composition comprising inhibin. This method utilizes a second animal's antibodies directed against a class of antibodies from a first animal as described above. Briefly described, the method comprises binding inhibin or the heterologous protein of the present invention, to a solid phase, and contacting the immobilized inhibin with a sample of blood from the animal to be tested. The sample is contacted with the immobilized inhibin under conditions wherein the inhibin will selectively interact with any anti-inhibin antibodies in the sample. After removing uninteracted antibodies from the sample and washing, a quantity of labeled antibodies from a second animal which are directed against a class of the first animal's antibodies are added. The labeled antibodies which are directed against the animal antibodies will then selectively interact with the antibodies which are bound to the immobilized inhibin. After removal of the uninteracted labeled antibodies, the presence or quantity of interacted labeled antibodies is determined by visualizing the label. Thus, the method detects the presence of antibodies directed against inhibin in the animal, and therefore determines if the animal has immunologically responded to the administration of a composition comprising inhibin.

It is to be understood that the method of determining if an animal has immunologically responded to the administration of a composition comprising inhibin can be performed on a female animal of any species. The animal can be a bird, mammal, or reptile, among others. More specifically, the mammal can be selected from the group consisting of, but not limited to, a cow, human, horse, cat, dog, sheep, mink, fox , otter, ferret, raccoon, and pig. The bird can be selected from, but is not limited to, an ostrich, emu, a rhea, and chicken. A preferred animal is a bird. A more preferred animal is a ratite. An especially preferred animal is an ostrich. Another preferred animal is an emu. Yet another preferred animal is a rhea.

One of ordinary skill in the art will understand that the immunoassay techniques that can be used in the above method are well known in the art. Therefore, any immunoassay technique, label, and visualization method can be used in the above method. A preferred immunoassay is ELISA, and a preferred label is horseradish peroxidase. Another preferred label is a colored latex bead. The colored latex bead can be any color desired for visualization purposes. Preferably, the latex bead is yellow, red, blue, or green. The colored latex bead can be hollow or solid, but it preferably is hollow to minimize its weight. The size of the latex bead varies according to its intended use in immunoassays. One of ordinary skill in the art would be able to ascertain the largest bead size that is visible yet does not interfere stearically with the immunoassay reactions. Preferably, the latex bead is less than 0.5µ in diameter, and most preferably it is less than 0.2µ in diameter.

Another method of determining if an animal has immunologically responded to the administration of a composition comprising inhibin wherein the immunoassay method is modified is as follows. Briefly described, the method comprises obtaining a sample of blood from an animal, and contacting it with labeled animal inhibin, or a fragment thereof. The sample is contacted with the labeled animal inhibin under conditions wherein the animal inhibin will selectively interact with any anti-inhibin antibodies in the sample. After removing uninteracted labeled inhibin from the sample, the presence or quantity of interacted labeled inhibin is determined by visualizing the label. The inhibin used in this method can be selected from: the fused heterologous inhibin protein of the present invention; endogenous inhibin, or fragments thereof; and exogenous inhibin, or fragments thereof.

This invention is further illustrated by the following examples, which are not to be construed in any way as imposing limitations upon the scope thereof. On the contrary, it is to be clearly understood that resort may be had to various other embodiments, modifications, and equivalents thereof which, after reading the description herein, may suggest themselves to those skilled in the art without departing from the scope of the appended claims.

### EXAMPLE 1

### Method for Producing a fused gene product comprising a gene encoded for expressing chicken inhibin, and a gene encoded for expressing maltose binding protein.

The following is a method for producing a fused gene product comprising a gene (cINA₅₁₅) encoded for expressing a fragment of alpha-subunit chicken inhibin (SEQ ID NO:2 or SEQ ID NO:4), and a gene encoded for expressing maltose binding protein. The fused gene product of the present invention is made from the pMAL™-c vector kit, from New England Biolabs, Beverly Massachusetts.

The pMAL™ vectors provide a method for expressing a protein expressed from a cloned gene or open reading frame. The cloned gene is inserted downstream from a malE gene, which encodes maltose-binding protein ("MBP"), and results in the expression of an MBP fusion protein ("MBP-cINA₅₁₅"). The method yields high-level expression of the cloned sequences, and a one-step purification for the fusion protein, MBP-cINA₅₁₅, using MBP's affinity for maltose.

The following is a Method of Ligating the Inhibin Gene, cINA₅₁₅, into a pMAL™-c Vector:
1. Digest 0.5 µg pMAL™-c plasmid DNA in 20 µl with chicken inhibin gene, cINA₅₁₅.
2. Digest 20 µg of λ DNA from the gt 11 clone in 200 µl with chicken inhibin gene, cINA₅₁₅.
3. Add 0.05 Units of calf intestinal alkaline phosphatase (NEB #290) to the vector DNA digestion. Incubate at 37°C for 1 hour.
4. Check for complete digestion by running 4µl of the pMAL™ reaction and 15 µl of the λ reaction on a 0.8% agarose gel. Add 1 µl and 8 µl of 0.5 M EDTA to the pMAL™ and λgt11 digestions, respectively.
5. Add an equal volume of a 1:1 phenol/chloroform mixture to the restriction digests, mix, and remove the aqueous (top) phase and place in a fresh tube. Repeat with chloroform alone.
6. Add 10 µg glycogen or tRNA to the vector digest as carrier. For both digests, add 1/10th volume 3 M sodium acetate, mix, then add an equal volume isopropanol. Incubate at room temperature for 10 minutes.
7. Microcentrifuge for 15 minutes. Pour off the supernatant, rinse the pellet with 70% ethanol, and allow to dry.
8. Resuspend each sample in 25 µl of 10 mM Tris-Cl, 1 mM EDTA, pH 8.0.
9. Mix: 2 µl vector digest; 6 µl insert digest; add 8 µl water, then heat the DNA mixture at 65°C for 5 minutes. Cool on ice, then add 4 ml 5x ligase buffer; 0.5 µl NEB T4 ligase (#202; ∼200 units); and incubate at 16°C for 2 hours to overnight.
10. Heat at 65°C for 5 minutes; cool on ice.
11. Mix with 25 µl competent TB1 (or any *lacZα*-complementing strain) and incubate on ice for 5 minutes. Heat to 42°C for 2 minutes.
12. Add 0.1 ml LB and incubate at 37°C for 20 minutes. Spread on an LB plate containing 100 µg/ml ampicillin. Incubate overnight at 37°C. Pick colonies with a sterile toothpick onto a master LB amp plate and an LB amp plate containing 80 µg/ml Xgal and 0.1 mM IPTG. Incubate at 37°C for 8 to 16 hours. Score the Lac phenotype on the Xgal plate and recover the "white" clones from the master.
13. Screen for the presence of inserts in one or both of the following ways:
   A. Prepare miniprep DNA. Digest with an appropriate restriction endonuclease to determine the presence and orientation of the insert.
   B.
      i) Grow a 5 ml culture in LB amp broth to about 2x 10⁸/ml.
      ii) Take a 1 ml sample. Microcentrifuge for 2 minutes, discard the supernatant and resuspend the cells in 50 µl protein gel SDS-PAGE sample buffer.
      iii) Add IPTG to the remaining culture to 0.3 mM, for example 15 µl of a 0.1 M stock solution. Incubate at 37°C with good aeration for 2 hours.
      iv) Take a 0.5 ml sample. Microcentrifuge for 2 minutes, discard the supernatant and resuspend the cells in 100 µl SDS-PAGE sample buffer.
      v) Boil the samples 5 minutes. Electrophorese 15 µl of each sample on a 10% SDS-PAGE gel along with a set of protein MW standards and 15 µl of the supplied MBP in SDS-PAGE sample buffer. Stain the gel with Coomassie brilliant blue. An induced band is easily visible at a position corresponding to the molecular weight of the fusion protein. The molecular weight of the MBP alone is 42,000 Daltons.

### EXAMPLE 2

### Method for Producing a fused heterologous protein, "MBP-cINA₅₁₅", comprising chicken inhibin and maltose binding protein.

The following is a method of producing a fused heterologous protein comprising chicken inhibin and maltose binding protein, "MBP-cINA₅₁₅". The fused gene product of Example 1 expresses the fused heterologous maltose binding protein-inhibin protein, "MBP-cINA₅₁₅", as follows:
1. Inoculate 80 ml rich broth + glucose and ampicillin (see *Media and Solutions* below) with 0.8 ml of an overnight culture of cells containing the fusion plasmid of Example 1.
2. Grow at 37°C with good aeration to 2 x 10⁸ cells/ml (A₆₀₀ of ∼0.5). Take a sample of 1 ml and microcentrifuge for 2 minutes (uninduced cells). Discard supernatant and resuspend the cells in 50 µl SDS-PAGE sample buffer. Vortex and place on ice.
3. Add IPTG (isopropylthiogalactoside) to the remaining culture to give a final concentration of 0.3 mM, e.g. 0.24 ml of a 0.1 M stock in H₂O (see *Media and Solutions).* Continue incubation at 37°C for 2 hours. Take a 0.5 ml sample and microcentrifuge for two minutes (induced cells). Discard supernatant and resuspend the cells in 100 µl SDS-PAGE: sample buffer. Vortex to resuspend cells and place on ice.
4. Divide the culture into two aliquots. Harvest the cells by centrifugation at 4000 x g for 10 min. Discard the supernatant and resuspend one pellet (sample A) in 5 ml of lysis buffer (see *Media and Solutions*). Resuspend the other pellet (sample B) in 10 ml 30 mM Tris-Cl, 20% sucrose, pH 8.0 (8 ml for each 0.1 g cells wet weight).
5. Freeze samples in a dry ice-ethanol bath (or overnight at -28°C). Thaw in cold water (20°C is more effective than 70°C, but takes longer).
6. Sonicate, monitor cell breakage, by measuring the release of protein using the Bradford assay or the release of nucleic acid at A_{260°} until it reaches a maximum. Add 0.6 ml 5M NaCl.
7. Centrifuge at 9,000 x g for 20 minutes. Decant the supernatant (crude extract 1) and save on ice. Resuspend the pellet in 5 ml lysis buffer. This is a suspension of the insoluble matter (crude extract 2).

*Column Purification of heterologous fused maltose binding protein-inhibin protein, "MBP-cINA*_{*515*}*", as produced above, is as follows:*
1. Swell amylose resin (1.5 g) for 30 min. in 50 ml column buffer (see *Media and Solutions)* in a 250 ml filter flask. De-gas with an aspirator. Pour in a 2.5 x 10 cm column. Wash the column with 3 column volumes of the same buffer + 0.25% Tween 20.
   The amount of resin needed depends on the amount of fusion protein produced. The resin binds about 3 mg/ml bed volume, so a column of about 15 ml should be sufficient for a yield of up to 45 mg fusion protein/liter culture. A 50 ml syringe plugged with silanized glass wool can be substituted for the 2.5 cm column. The column height to diameter ratio should be less than or equal to 4.
2. Dilute the crude extract 1:5 with column buffer + 0.25% Tween 20. Load the diluted crude extract at a flow rate of [10x (diameter of column in cm)²]ml/hr. This is about 1 ml/min. for a 2.5 cm column.
   The dilution of the crude extract is aimed at reducing the protein concentration to about 2.5 mg/ml. If the crude extract is less concentrated, do not dilute it as much. A good rule of thumb is that 1 g wet weight of cells gives about 120 mg protein.
3. Wash with 2 column volumes column buffer + 0.25% Tween 20.
4. Wash with 3 column volumes column buffer without Tween 20.
5. Elute the fusion protein, "MBP-cINA₅₁₅", with column buffer + 10 mM maltose + 0.1% SDS (optional 10 mM β-mercaptoethanol, 1 mM EGTA). Collect 10-20 3 ml fractions. Assay the fractions for protein, e.g., by the Bradford assay or A₂₆₀°; the fractions containing the fusion protein have easily detectable protein. The fusion protein elutes soon after the void volume of the column.

### Media and Solutions

* Rich medium + glucose and ampicillin= per liter: 10g tryplone, 5g yeast extract, 5g NaCl, 2g glucose. Autoclave; add sterile ampicillin to 100 µg/ml.
* 0.1 M IPTG Stock= 1.41g IPTG (isopropyl-β-o-thiogalactoside); add H₂O to 50 ml. Filter, and sterilize.
* 0.5 M sodium phosphate buffer, pH 7.2 (stock)=
   (A) 69.0 g NaH₂PO₄H₂O to 1 liter with H₂O.
   (B) 70.9 g Na₂HPO₄ to 1 liter with H₂O.
   Mix 117 ml (A) with 383 ml (B). The pH of this stock should be ∼7.2. Diluted to 10 mM in column buffer, the pH should be 7.0.

| *Lysis Buffer* | |
|---|---|
| **Per Liter** | **Final Concentration** |
| 20 ml 0.5M Na₂HPO₄ | 10 mM phosphate |
| 1.75 g NaCl | 30 mM NaCl |
| 10 ml 25% Tween 20 | 0.25% Tween j20 |
| 0.7 ml β-mercaptoethanol ("β-ME") (optional) | 10 mM β-ME |
| 20 ml 0.5M EDTA (pH 8) | 10 mM EDTA |
| 10 ml 1M EGTA (pH 7) | 10 mM EGTA |
| Adjust to pH 7.0 with HCL or NaOH | |

| *Column Buffer* | |
|---|---|
| **Per Liter** | **Final Concentration** |
| 20 ml 0.5M sodium phosphate, pH 7.2 | 10 mM phosphate |
| 29.2 g NaCl | 0.5 M NaCl |
| 1 ml 1M sodium azide | 1 mM azide |
| 0.7 mM β-ME (optional) | 10 mM β-ME |
| 1 ml 1M EGTA (pH 7) (optional) | 1 mM EGTA |
| Adjust to pH 7.0 if necessary. | |

| *Low Salt Column Buffer* | |
|---|---|
| Per Liter | Final Concentration |
| 20 ml 0.5M sodium phosphate, pH 7.2 | 10 mM phosphate |
| 1.75 NaCl | 30 mM NaCl |
| 1 ml 1M sodium azide | 1 mM azide |
| 0.7 ml β-mercaptoethanol (optional) | 10 mM β-ME |
| 1 ml 1 M EGTA (pH 7) (optional) | 1 mM EGTA |
| Adjust to pH 7.0 if necessary. | |

The purity of the fused chicken inhibin-MBP heterologous protein, "MBP-cINA₅₁₅", after passing through the column is illustrated in Figure 1, columns "E". The column marked "F" is the eluent from the column when no heterologous protein has been loaded on the column (the negative control). The columns marked "B" represent the plasmid pMAL™-c vector standards. The columns marked "C" are molecular weight standards. The columns marked "D" are the actual pMAL™-c vector used in the preparation of the fused chicken-inhibin-MBP heterologous protein, "MBP-cINA₅₁₅", prior to the insertion of the inhibin gene as described in Example 2. The above proteins were electrophoresed on a SDS-PAGE gel in SDS-PAGE sample buffer, and stained with Coomassie brilliant blue stain.

### EXAMPLE 3

### Method for Immunizing an Ostrich against Inhibin.

The following is a method for immunizing an ostrich against inhibin. A primary immunization is administered to the ostrich approximately six months prior to the bird's first breeding season, and then booster immunizations are administered at one month intervals for six months. Accordingly, it is preferable to administer the primary immunization to an ostrich when it is between approximately 18 and 24 months old. The primary immunization comprises between approximately 1.5 to 3.0 mg of a fused heterologous protein comprising chicken inhibin (a fragment of the alpha subunit) and maltose binding protein produced by the methods described in Examples 1 and 2. The booster immunizations comprise between approximately 0.75 to 1.5 mg of the fused heterologous protein. The fused heterologous protein is emulsified in Freunds Complete Adjuvant (Sigma Chemical Co., St. Louis, MO) in the primary immunization, and the fused heterologous protein is emulsified in Freuds Incomplete Adjuvant (Sigma) in the booster immunizations. The fused heterologous protein composition is injected subcutaneously at three sites along the upper thigh region of the ostrich.

### EXAMPLE 4

### Method for producing ostrich antibodies selectively directed against inhibin.

The following is a method of producing ostrich antibodies ("ostrich anti-chicken inhibin antibodies") which are selectively directed against a heterologous protein of the present invention comprising chicken inhibin and maltose binding protein. More particularly, the ostrich antibodies are IgG antibodies. The heterologous protein used is a fused protein comprising chicken inhibin and maltose binding protein produced by the methods described in Examples 1 and 2. To produce the antibodies, immunize an ostrich with chicken inhibin-MBP heterologous protein according the method described in Example 3. The amount administered to the ostrich must be sufficient to elicit an immunological response in the ostrich against the heterologous protein. Withdraw approximately 5ml of blood from the ostrich, and then isolate antibodies directed against inhibin from the remainder of the blood sample. Any separation method known in the art can be used to isolate the antibodies. Preferably, standard ELISA techniques are employed.

### EXAMPLE 5

### Method of producing goat antibodies selectively directed against ostrich antibodies.

The following is a method of producing goat antibodies which are selectively directed against a class of ostrich antibodies, including the antibodies produced in Example 4, more particularly IgG ostrich antibodies. In this method a goat is immunized with 0.5 to 3.0 mg ostrich IgG such that an immunological response occurs in the goat against the ostrich IgG. The ostrich IgG are obtained from a pool of sera from different ostriches. Blood is obtained from the goat, and goat anti-ostrich IgG is then isolated from the sample using standard techniques that are well known in the art.

The pool of sera is purified using standard methods that involve precipitation using 50% ammonium sulfate, and subsequent fractionation using a protein-A sepharose column. Preferably, the IgG precipitation is conducted as follows. Twelve milliliters of sera containing IgG is diluted 1:1 with 50 mM-Tris (pH 8.0). Next, 24 ml of saturated ammonium sulphate is added slowly with stirring (all at 4°C), and the mixture is stirred for approximately 2 hours. Centrifuge the mixture at 10,000 rpm for 10 minutes to collect the precipitate. Resuspend the precipitate in 50 mM-Tris/Saline (to 12 ml.), and dialyse overnight versus 2L of 50mM-Tris at 4°C, the final volume being 20 ml.

Preferably, the subsequent fractionation using a protein-A sepharose column is as follows. The column contains protein-A sepharose CL-4B (Pharmacia Biotech, Inc., Piscataway, NJ). The column is loaded with approximately 5 milliliters of ammonium sulfate/serum precipitate. The sample is allowed to bind to the protein A-sepharose for approximately 30 minutes. Next, the column is washed with 0.1M phosphate buffer (pH 7.5), and the absorbed IgG is eluted with 0.1M Glycine (pH 2.8). Finally, the eluted fractions are neutralized by adding a few drops of 1M Tris-HCL (pH 9). The quality of the purified ostrich IgG is tested by visualization after SDS-polyacrylamide gel electrophoresis before it is administered to a goat.

The method of immunization and the adjuvants used are not critical to the invention, thus any method known in the art can be used, and any adjuvant system known in the art can be used. Preferably, the purified ostrich IgG will be injected into a goat subcutaneously. It is also preferable to administer boost injections at four week intervals using Freund's incomplete adjuvant. Preferably, the purified IgG will be administered with Freund's Complete Adjuvant or Hunter's Titermax (Sigma Chemical Co., St. Louis, Missouri). A goat develops a satisfactory immune response after three to four injections.

Next, 5-10 ml of blood is withdrawn from the goat, and the goat antibodies directed against ostrich IgG are isolated from the blood sample. Any method known in the art can be used to separate the goat antibodies from the blood sample. A preferred method for separating the goat antibodies from the sample is by passing the blood sample through an ostrich IgG column. The goat antibodies are then collected from the column by washing the column with glycine buffer-pH 8.0.

### EXAMPLE 6

### Method of monitoring the immunological response of an ostrich after its vaccination with a fused heterologous protein.

The following is a method of monitoring the immunological response of an ostrich after it has been vaccinated with a fused heterologous protein comprised of chicken inhibin and maltose binding protein produced by the method of Examples 1 and 2, wherein the immunological response is monitored using the goat antibodies produced in Example 5. To determine whether an ostrich has immunologically reacted to the fused inhibin-MBP heterologous protein, first bind the heterologous protein to a solid phase. Next, withdraw 5-10 ml of blood from an ostrich that has been immunized with the heterologous protein, and isolate the serum from the blood. Contact the immobilized heterologous protein with the serum under conditions wherein the heterologous protein will selectively interact with any anti-inhibin antibodies in the serum. After washing, add the goat antibodies produced in Example 5 which have been labeled with HRP. The labeled goat antibodies will then selectively interact with the ostrich antibodies which are bound to the immobilized heterologous protein. After the removal of the uninteracted labeled antibodies, the presence or quantity of interacted HRP labeled goat antibodies is determined by visualizing the label. Preferably, the label is visualized by adding Nitro Blue Tetrazolium ("NBT"), a substrate to HRP.

One of ordinary skill in the art will understand that the immunoassay techniques that are used in the above method are well known in the art. Therefore, any immunoassay technique, label, and visualization method can be used in the above method. A preferable immunoassay is ELISA, and a preferable label is horseradish peroxidase. Another preferable label is a colored latex bead.

### EXAMPLE 7

### Method for determining the reproductive potential of an ostrich.

The following is a method for determining the reproductive potential of an ostrich by quantifying the amount of inhibin in the ostrich's blood. Circulating inhibin concentrations in the blood of an ostrich can be determined using standard sandwich ELISA techniques. First, bind the anti-inhibin antibodies produced in Example 4, to the wells of a microtiter plate. After washing and blocking the plate, then add a quantity of blood plasma or serum that was obtained from the ostrich to a well of the microtiter plate. After allowing any inhibin in the sample, if present, to selectively interact with the immobilized anti-inhibin antibodies, the sample is washed from the well of the plate. Next, add a different anti-inhibin antibody to the well than that produced in Example 4, which is conjugated with horseradish peroxidase. The HRP conjugated anti-inhibin antibody differs from the immobilized anti-inhibin body in that they are selectively directed against different portions of inhibin. After allowing the labeled anti-inhibin antibody to selectively interact with any immobilized inhibin, any uninteracted labeled anti-inhibin antibodies are removed by washing. The amount of inhibin present in the plasma sample is determined by adding NBT to the well and visualizing the amount of immobilized labeled anti-inhibin antibody in the well. Standard positive and negative controls are run simultaneously in neighboring plate wells. Standard positive and negative controls are to be run simultaneously in neighboring plate wells. Many immunoassay techniques, labels and visualization methods are well known in the art. Accordingly, any immunoassay method, label, and visualization technique can be used in the present invention.

### EXAMPLE 8

### Method for accelerating the onset of egg lay in Quail.

As stated above, the chicken inhibin a-subunit cDNA clone (cINA6) inserted into the EcoR 1 site of Bluescript was obtained as a gift of P. A. Johnson (Cornell University). A DNA fragment ("cINA₅₁₅") was excised from the cINA6 clone using Pst I digestion. The cINA₅₁₅ DNA fragment encompassed most of the mature chicken inhibin α-subunit. This fragment (cINA₅₁₅) was cloned in plasmid p-MALTM-c in frame with the maltose binding protein ("MBP") and a fusion protein of appropriate size (Lane E; Figure 1) was detected after IPTG (isopropyl β-D-thiogalactopyranoside) induction and SDS-PAGE. The resulting protein conjugate ("MBP-cINA₅₁₅") was used as an antigen to immunize pre-pubescent, female Japanese quail (*Coturnix coturnix japonica)* against circulating inhibin levels as described below.

Hatchling quail were brooded in a Model 2S-D Petersime brooder battery modified for quail. Initial brooding temperature was approximately 37.8 C with a weekly decline of approximately 2.8 C until ambient temperature was achieved. During the growing period *(ie.,* until approximately 6 wks-of-age), a quail starter ration (28% CP, 2,800 kcal ME/kg of feed) and water were provided for *ad libitum* consumption, and continuous dim light (22 lx) with a 14 h light (280 to 300 lx):10 hr dark override was used. At 25 days-of-age, 100 quail were randomly and equally assigned to one of four injection groups (25 birds per group) as follows: (1) MBP-cINA₅₁₅ in a polydispersed β-(1,4) linked acetylated mannan ("Acemannan"), an immunostimulant vehicle (Chinnah et al., *Antigen dependent adjuvant activity of a polydispersed β-(1,4)-linked acetylated mannan (acemannan),* Vaccine, Vol. 10, Issue 8, 1992, p. 551-557) ("MBP-cINA₅₁₅/ACE"), (2) MBP-cINA₅₁₅ in Freund's adjuvant ("MBP-cINA₅₁₅/FRN"), (3) Acemannan (immunostimulant control; "ACE"), or (4) Freund's (adjuvant control; "FRN"). Birds immunized against inhibin (Groups 1 and 2) were given approximately 0.75 mg MBP-cINA₅₁₅ per bird in the appropriate control vehicle. Equivalent vehicular injection volumes (0.2 mL) of ACE or FRN were used in Groups 1 and 3 and Groups 2 and 4, respectively. All injections were given subcutaneouly using tuberculin syringes fitted with 25 ga needles. Following the initial injections, quail were wingbanded to identify them by treatment before housing (individually) in laying cages. Weekly booster inhibin immunizations of approximately 0.375 mg MBP-cINA₅₁₅ per bird, or appropriate control challenges, were subsequently administered for five consecutive weeks (*ie.,* at 32, 39, 46, 53, and 60 days-of-age). Beginning at 6 wk-of-age, a quail breeder ration (21% CP, 2,750 kcal ME/kg of feed) and water were provided for *ad libitum* consumption.

Beginning at 41 days-of age (considered Day 1 of the egg lay cycle), daily hen-day egg production ("HDEP") and mortality ("MORT") measures were recorded for 12 consecutive weeks. In addition, average age at first egg lay ("FIRST") and age at which hens reached 50% egg production ("FIFTY") were calculated for each of the four treatment groups.

Hen-day egg production data were subjected to two different analyses of variance ("ANOVAs"), each of which incorporated a completely randomized design with a split-plot arrangement of treatments. In the first ANOVA, the main plot consisted of the four injection treatments (MBP-cINA₅₁₅/ACE, MBP-cINA₅₁₅/FRN, ACE, or FRN) and the 12 laying periods of 7 days each comprised the split. In the second ANOVA, to more powerfully test the effect of inhibin immunoneutralization versus control response, data from the two inhibin treatments were pooled and data from the two control treatments were pooled. Thus, the main plot consisted of two injection treatments (pooled MBP-cINA₅₁₅/ACE and MBP-cINA₅₁₅/FRN data versus pooled ACE and FRN data) with the 12 laying periods of 7 days each comprising the split.

To date, information is preliminary as only 35 days of data have been collected. Cumulative percent HDEP rates for weekly intervals of egg lay (days 0, 7, 14, 21, and 35) during the first 35 days of study are plotted in Figure 2 (wherein the four injection groups were considered as separate treatments) and Figure 3 (wherein only non-immunized (control) versus inhibin-immunized group comparisons were made). Mean HDEP rates during Week 1, Week 2, Week 3, Week 4, Week 5 and Weeks 1 through 5 combined for the four injection groups considered as independent treatments are given in Table 2.

Table 3 gives mean HDEP rates during the same timeframes when data were pooled for direct control versus immunized treatment comparisons. Table 4 contains FIRST egg-lay data for both of the statistical scenarios considered (*ie.,* the four injection treatment groups and the pooled data comparisons).

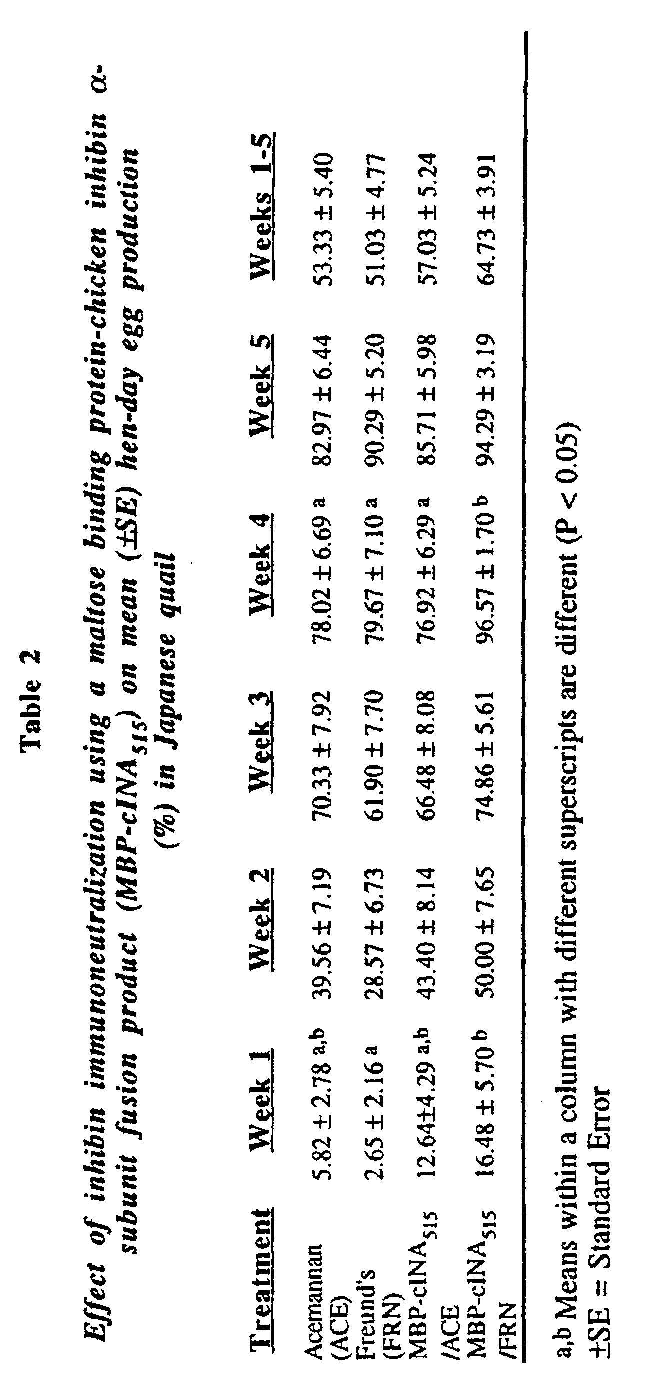

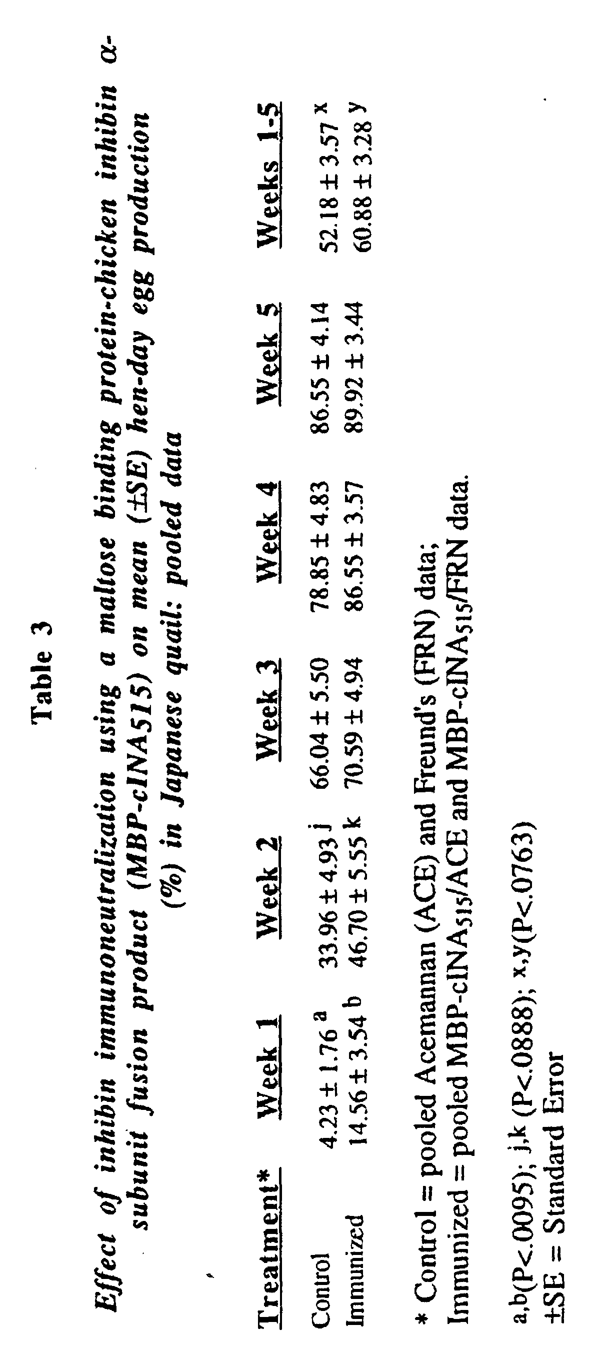

**Table 4**

| ***Effect of inhibin immunoneutralization using a maltose binding protein-chicken inhibin α-subunit fusion product (MBP-cINA***_{***515***}***) on mean (±SE) age at first egg lay (FIRST) in Japanese quail*** | |
|---|---|
| **Treatment*** | **FIRST (days-of-age)** |
| Acemannan (ACE) | 54.92 ± 1.77 a,b |
| Fruend's (FRN) | 55.73 ± 1.58 ^{a} |
| MBP-cINA₅₁₅/ACE | 53.58 ± 1.81 ^{a,b} |
| MBP-cINA₅₁₅/FRN | 50.38 ± ^{b} |
| | |
| Control | 55.33 ± 1.17 ^{x} |
| Immunized | 51.98 ± 1.07 ^{y} |

| | |
|---|---|
| * Control = pooled Acemannan (ACE) and Fruend's (FRN) data; Immunized = pooled MBP-cINA₅₁₅/ACE and MBP-cINA₅₁₅/FRN data | |
| ^{a,b}(P<.05); ^{x,y}(P<.0373) SE= Standard Error | |

Thus far, mortality has not been a factor as only four birds have died (three controls, one treated). Such mortality (4 %) would be well within expected limits for quail that have reached 76 days-of-age. In addition, incidences of shelless, thin shelled, and mishapened eggs were low, well within normal frequencies of occurrence, and evenly distributed among the four injection treatment groups (*ie.,* inhibin immunization did not alter numbers of defective eggs laid).

An initial positive effect of inhibin immunoneutralization on production performance was extant. This effect was particularly evident in the pooled HDEP (Table 3) and FIRST (Table 4) data sets where increased numbers of observations comprising means augmented power of the statistical tests applied.

The data shows that onset of puberty was accelerated in the inhibin treatment groups. This was evidenced in both the HDEP and FIRST data collected. There was a high degree of statistical confidence (P<.0095) associated with the higher mean HDEP rate found in inhibin-treated quail during Week 1 of the study, a difference that was less evident (P<.0888) during Week 2 (Table 3). This marginal treatment group difference observed during Week 2 may represent that egg lay, although delayed, was beginning to intensify in control-injected quail (See Figure 2). Consequently, the strength of the statistical difference found between the two treatment groups (inhibin versus no-inhibin) during Week 1 would expectedly become diluted with advancing time of lay. Indeed, when data from Weeks 1 through 5 were combined, a significantly higher mean HDEP rate of intermediate statistical relevance (P<.0763) was found in inhibin-treated hens.

Also, the mean age at which immunized quail (51.98 days-of-age) laid their first egg (FIRST) was advanced (P<.0373) by 3+ days when compared to non-immunized quail (55.33 days-of-age) (Table 4). This effect of inhibin immunization on acceleration of egg lay was particularly noteworthy in MBP-cINA₅₁₅/FRN-treated quail which had an average mean FIRST of 50.38 days-of-age (Table 4).

### EXAMPLE 9

### Method for accelerating the onset of egg lay in Ostriches.

The protein conjugate (MBP-cINA₅₁₅) is used as an antigen to immunize prepubescent, female ostriches against circulating inhibin levels, and to therefore accelerate the onset of egg lay in the treated ostriches. The method described in Example 8 is followed with the following exceptions. The average age at puberty for untreated ostriches is between approximately 28 and 32 months. The following is the treatment schedule for ostriches having an approximate body weight range of 150 to 300 pounds: primary (first) injection of 5.0 mg of the heterologous protein of the present invention on its 26th month of age; and boosters of 2.5 mg on the 27th, 28th, 30th, 32nd, 34th, and 36th month of age.

### EXAMPLE 10

### Method for accelerating the onset of egg lay in Emu.

The protein conjugate (MBP-cINA₅₁₅) is used as an antigen to immunize prepubescent, female emu against circulating inhibin levels, and to therefore accelerate the onset of egg lay in the treated emu. The method described in Example 8 is followed with the following exceptions. The average age at puberty for untreated emu is approximately 20 months. The following is the treatment schedule for emu having an approximate body weight range of 50 to 90 pounds: primary (first) injection of 3.0 mg of the heterologous protein of the present invention on its 18th month of age; and boosters of 1.5 mg on the 19th, 20th, 22nd, 24th, 26th, and 30th month of age.

### EXAMPLE 11

### Method for accelerating the onset of egg lay in Chickens.

The protein conjugate (MBP-cINA₅₁₅) is used as an antigen to immunize prepubescent, female chickens against circulating inhibin levels, and to therefore accelerate the onset of egg lay in the treated chickens. The method described in Example 8 is followed with the following exceptions. The average age at puberty for an untreated chicken is approximately 20 weeks. The following is the treatment schedule for a chicken having an approximate body weight range of 2.0 to 3.5 pounds: primary (first) injection of 1.5 mg of the heterologous protein of the present invention on its 15th week of age; and boosters of 0.75 mg on the 17th, 20th, 24th, 30th, 40th, and 50th week of age.

### EXAMPLE 12

### Method for accelerating the onset of egg lay in Turkeys.

The protein conjugate (MBP-cINA₅₁₅) is used as an antigen to immunize prepubescent, female turkeys against circulating inhibin levels, and to therefore accelerate the onset of egg lay in the treated turkeys. The method described in Example 8 is followed with the following exceptions. The average age at puberty for an untreated turkey is approximately 30 weeks. The following is the treatment schedule for a turkey having an approximate body weight range of 9.0 to 12 pounds: primary (first) injection of 2.0 mg of the heterologous protein of the present invention on its 28th week of age; and boosters of 1.0 mg on the 29th, 30th, 34th, 38th, 46th, and 54th week of age.

### EXAMPLE 13

### Method for accelerating the onset of egg lay in Parrots.

The protein conjugate (MBP-cINA₅₁₅) is used as an antigen to immunize prepubescent, female parrots against circulating inhibin levels, and to therefore accelerate the onset of egg lay in the treated parrots. The method described in Example 8 is followed with the following exceptions. The average age at puberty for an untreated parrot is approximately 30 months. The following is the treatment schedule for a parrot having an approximate body weight range of 0.5 to 1.25 pounds: primary (first) injection of 0.75 mg of the heterologous protein of the present invention on its 28th month of age; and boosters of 0.375 mg on the 29th, 30th, 32nd, 34th, 36th, and 38th month of age.

It should be understood, of course, that the foregoing relates only to preferred embodiments of the present invention and that numerous modifications or alterations may be made therein without departing from the scope of the invention as set forth in the appended claims.

## Claims

1. A method which increases egg production of a bird, comprising administering an effective amount of a protein comprising alpha-subunit avian inhibin protein, or a fragment thereof, and a carrier protein, to the bird.

2. The method of claim 1, wherein the bird is selected from the group consisting of ratites, psittaciformes, falconiformes, piciformes, strigiformes, passeriformes, coraciformes, ralliformes, cuculiformes, columbiformes, galliformes, anseriformes, and herodiones.

3. The method of claim 2, wherein the ratite is an ostrich, emu, rhea, kiwi or cassowary.

4. The method of claim 1, wherein the bird is a chicken, turkey, parrot, parakeet, macaw, falcon, eagle, quail, hawk, pigeon, cockatoo, song bird, jay bird, blackbird, finch, warbler, goose, duck, or sparrow.

5. The method of claim 4, wherein the bird is a chicken or turkey.

6. The method of any of claims 1 to 5, wherein the carrier protein is maltose binding protein, bovine serum albumin, ovalbumin, flagellin, keyhole-limpet hemocyanin, serum albumin, gamma globulin or polymers of amino acids.

7. The method of any of claims 1 to 6, wherein the alpha-subunit avian inhibin protein, or a fragment thereof, comprises the amino acid sequence set forth in SEQ ID NO:2 or SEQ ID NO:4.

8. The method of any of claims 1 to 7, wherein increase of egg production comprises accelerating onset of egg lay, increasing lifetime total egg lay, increasing rate of egg production or accelerating onset of puberty.

9. The method of any of claims 1 to 8, wherein the administration of an effective amount of the protein comprises an initial immunization followed by one or more booster injections.

10. The method of any of claims 1 to 9, wherein the administration of the protein occurs before, during and/or after puberty.

11. A fused protein comprising alpha-subunit avian inhibin protein or a fragment thereof, fused to a carrier protein.

12. The fused protein of claim 11, wherein the alpha-subunit avian inhibin protein or a fragment thereof, comprises the amino acid sequence set forth in SEQ ID NO:2 or SEQ ID NO:4.

13. The fused protein of claims 11 or 12, wherein the carrier protein is maltose binding protein, bovine serum albumin, ovalbumin, flagellin, keyhole limpet hemocyanin, serum albumin, gamma globulin or polymers of amino acids.

14. A method of producing a fused protein according to any of claims 11 to 13, comprising the steps of:
a) providing double strand cDNA which is encoded for alpha-subunit avian inhibin protein or a fragment thereof
b) providing a vector which has coding information for the production of a carrier protein
c) ligating the alpha-subunit avian inhibin cDNA into the vector
d) inserting the ligated vector into an expression system, and
e) expressing a fused protein comprising alpha-subunit avian inhibin protein or a fragment thereof and a carrier protein.

15. The method of claim 14, wherein the alpha-subunit avian inhibin cDNA comprises the sequence of SEQ ID NO:1 or SEQ ID NO:3.

16. A fusion gene product encoding a gene for the expression of alpha-subunit avian inhibin protein, or a fragment thereof, fused to a gene encoded for the expression of a carrier protein.

17. The fusion gene product of claim 16, wherein the gene encoded for the expression of the alpha-subunit avian inhibin protein or the fragment thereof is encoded for the production of a inhibin protein, or the fragment thereof, comprising the amino acid sequence set forth in SEQ ID NO:2 or SEQ ID NO:4.

18. The fusion gene product of claim 16, comprising the sequence of SEQ ID NO:1 or SEQ ID NO:3.

19. The fusion gene product of any of claims 16 to 18, wherein the gene encoded for the expression of the carrier protein is encoded for the production of a protein selected from maltose binding protein, bovine serum albumin, ovalbumin, flagellin, keyhole-limpet hemocyanin, serum albumin, gamma globulin or polymers of amino acids.

20. A vector, comprising a DNA sequence according to the fusion gene of any of claims 16 to 19.

21. A cell, containing the vector of claim 20.

## Patentansprüche

1. Ein Verfahren, das die Eiproduktion eines Vogels erhöht, umfassend ein Verabreichen einer effektiven Menge eines Proteins an den Vogel, wobei das Protein die α-Untereinheit eines Vogel-Inhibinproteins, oder ein Fragment davon und ein Trägerprotein umfasst.

2. Das Verfahren nach Anspruch 1, wobei der Vogel ausgewählt ist aus der Gruppe, bestehend aus Ratitae, Psittaciformes, Falconiformes, Piciformes, Strigiformes, Passeriformes, Coraciiformes, Ralliformes, Cuculiformes, Columbiformes, Galliformes, Anseriformes und Herodiones.

3. Das Verfahren nach Anspruch 2, wobei der Ratita ein Vogel Strauß, Emu, Nandu, Kiwi oder Kasuar ist.

4. Das Verfahren nach Anspruch 1, wobei der Vogel ein Huhn, Truthahn, Papagei, Sittich, Ara, Falke, Adler, Wachtel, Habicht, Taube, Kakadu, Singvogel, Eichelhäher, Amsel, Fink, Grasmücke, Gans, Ente oder Sperling ist.

5. Das Verfahren nach Anspruch 4, wobei der Vogel ein Huhn oder Truthahn ist.

6. Das Verfahren gemäß einem der Ansprüche 1 bis 5, wobei das Trägerprotein Maltosebindeprotein, Rinderserumalbumin, Ovalbumin. Flagellin, Hämocyanin der Schlüsselloch-Napfschnecke, Serumalbumin, Gammaglobulin oder Polymere aus Aminosäuren ist.

7. Das Verfahren gemäß einem der Ansprüche 1 bis 6, wobei das α-Untereinheit-Vogel-Inhibinprotein, oder ein Fragment davon die Aminosäuresequenz umfasst, die in SEQ ID NO:2 oder SEQ ID NO:4 dargelegt ist.

8. Das Verfahren gemäß einem der Ansprüche 1 bis 7, wobei die Erhöhung der Eiproduktion die Beschleunigung des Beginns des Eierlegens, die Erhöhung des Gesamteierlegens während der Lebenszeit, die Erhöhung der Rate der Eierproduktion oder die Beschleunigung des Beginns der Pubertät einschließt.

9. Das Verfahren gemäß einem der Ansprüche 1 bis 8, wobei das Verabreichen einer effektiven Menge des Proteins eine initiale Immunisierung, gefolgt von einer oder mehreren Verstärkungsinjektionen umfasst.

10. Das Verfahren gemäß einem der Ansprüche 1 bis 9, wobei das Verabreichen des Proteins vor, während und/oder nach der Pubertät erfolgt.

11. Ein Fusionsprotein, enthaltend ein α-Untereinheit-Vogel-Inhibinprotein oder ein Fragment davon, fusioniert an ein Trägerprotein.

12. Das Fusionsprotein gemäß Anspruch 11, wobei das α-Untereinheit-Vogel-Inhibinprotein oder das Fragment davon die Aminosäuresequenz enthält, die in SEQ ID NO:2 oder SEQ ID NO:4 dargelegt ist.

13. Das Fusionsprotein der Ansprüche 11 oder 12, wobei das Trägerprotein Maltosebindeprotein, Rinderserumalbumin, Ovalbumin, Flagellin, Hämocyanin aus der Schlüsselloch-Napfschnecke, Serumalbumin, Gammaglobulin oder Polymere aus Aminosäuren ist.

14. Ein Verfahren zur Herstellung eines Fusionsproteins gemäß einem der Ansprüche 11 bis 13, enthaltend die Schritte:
a) Bereitstellen doppelsträngiger cDNA, die für ein α-Untereinheit-Vogel-Inhibinprotein oder ein Fragment davon kodiert,
b) Bereitstellen eines Vektors, der kodierende Informationen für die Herstellung eines Trägerproteins enthält,
c) Ligieren der α-Untereinheit-Vogel-Inhibin-cDNA in den Vektor,
d) Einbringen des ligierten Vektors in Expressionssystem, und
e) Expremieren eines Fusionsproteins, enthaltend ein α-Untereinheit-Vogel-Inhibinprotein oder ein Fragment davon und ein Trägerprotein.

15. Das Verfahren nach Anspruch 14, wobei die α-Untereinheit-Vogel-Inhibin-cDNA die Sequenzen von SEQ ID NO:1 oder SEQ ID NO:3 enthält.

16. Ein Fusionsgenprodukt, das ein Gen für die Expression von α-Untereinheit-Vogel-Inhibinprotein oder ein Fragment davon kodiert, fusioniert an ein Gen, das die Expression eines Trägerproteins kodiert.

17. Das Fusionsgenprodukt nach Anspruch 16, wobei das Gen, das für Expression des α-Untereinheit-Vogel-Inhibinproteins oder dem Fragment davon kodiert für die Produktion eines Inhibinproteins oder eines Fragments davon kodiert, das die Aminosäuresequenz enthält, die in SEQ ID NO:2 oder SEQ ID NO:4 dargelegt ist.

18. Das Fusionsgenprodukt gemäß Anspruch 16, enthaltend die Sequenz SEQ ID NO:1 oder SEQ ID NO:3.

19. Das Fusionsgenprodukt gemäß einem der Ansprüche 16 bis 18, wobei das Gen, das für die Expression des Trägerproteins kodiert, für die Produktion eines Proteins kodiert, das ausgewählt ist aus Maltosebindeprotein, Rinderserumalbumin, Ovalbumin, Flagellin, Hämocyanin der Schlüsselloch-Napfschnecke, Serumalbumin, Gammaglobulin oder Polymeren aus Aminosäuren.

20. Ein Vektor, enthaltend eine DNA-Sequenz entsprechend dem Fusionsgen aus einem der Ansprüche 16 bis 19.

21. Eine Zelle, enthaltend den Vektor aus Anspruch 20.

## Revendications

1. Procédé pour augmenter la production d'oeufs chez un oiseau qui comprend l'administration d'une quantité efficace d'une protéine incluant la sous-unité alpha de la protéine inhibine aviaire, ou un fragment de celle-ci et une protéine support, à l'oiseau.

2. Procédé selon la revendication 1,
dans lequel
l'oiseau est choisi dans le groupe constituant en oiseaux coureurs (ratites), en psittaciformes, en falconiformes, piciformes, strigiformes, en passériformes, en coraciformes, en ralliformes, en cuculiformes, en colombiformes, en galliformes (gallinacés), en ansériformes, et en hérodiones (échassiers).

3. Procédé selon la revendication 2,
dans lequel
le ratite est une autruche, un émeu, un nandou, un kiwi, ou un casoar.

4. Procédé selon la revendication 1,
dans lequel
l'oiseau est un poulet, une dinde, un perroquet, une perruche, un ara, un faucon, un aigle, une caille, un vautour, un pigeon, un cacaotés, un oiseau chanteur, un geai, un merle, un bouvreuil, une fauvette, une oie, un canard ou un moineau.

5. Procédé selon la revendication 4,
dans lequel
l'oiseau est un poulet ou une dinde.

6. Procédé selon l'une quelconque des revendications 1 à 5,
dans lequel
la protéine support est une protéine liant le maltose, la sérum albumine bovine, l'ovalbumine, la flagelline, l'hémocyanine de patelle à trou central, la sérum albumine, la gamma globuline, ou des polymères d'amino acides.

7. Procédé selon l'une quelconque des revendications 1 à 6,
dans lequel
la sous-unité alpha de la protéine inhibine aviaire ou un fragment de celle-ci inclut la séquence d'amino acides exposée dans la séquence SEQ ID n°2 ou SEQ ID n°4.

8. Procédé selon l'une quelconque des revendications 1 à 7,
dans lequel
l'augmentation de la production d'oeufs comprend l'accélération du démarrage de la ponte d'oeufs, l'augmentation de la durée de vie totale de la ponte d'oeufs, l'augmentation de la vitesse de production d'oeufs ou l'accélération du démarrage de la puberté.

9. Procédé selon l'une quelconque des revendications 1 à 8,
dans lequel
l'administration d'une quantité efficace de la protéine inclut une immunisation initiale suivie par une ou davantage d'injections de rappel.

10. Procédé selon l'une quelconque des revendications 1 à 9,
dans lequel
l'administration de la protéine survient avant, pendant et/ou après la puberté.

11. Protéine fusionnée contenant de la sous-unité alpha de la protéine inhibine aviaire ou un fragment de celle-ci fusionnée avec une protéine vecteur.

12. Protéine fusionnée selon la revendication 11,
dans laquelle
la sous unité alpha de la protéine inhibine aviaire ou un fragment de celle-ci comprend la séquence d'amino acides exposé dans SEQ ID n°2 ou SEQ ID n°4.

13. Protéine fusionnée des revendications 11 ou 12,
dans laquelle
la protéine vecteur est une protéine liant le maltose, la sérum albumine bovine, l'ovalbumine, la flagelline, l'hémocyanine, la gamma globuline ou les polymères d'aminoacides.

14. Procédé de production d'une protéine fusionnée selon l'une quelconque des revendication 11 à 13,
qui comprend les étapes de :
a) fournir l'ADNc à double brin qui est encodé pour la sous-unité alpha de la protéine inhibine aviaire ou un fragment de celle-ci,
b) fournir un vecteur qui a des informations de codage pour la production d'une protéine vecteur,
c) ligaturer l'ADNc pour la sous-unité alpha de l'inhibine aviaire dans le vecteur,
d) insérer le vecteur ligaturé dans un système d'expression et
e) exprimer une protéine fusionnée comprenant la sous-unité alpha de la protéine inhibine aviaire ou un fragment de celle-ci et une protéine vecteur.

15. Procédé selon la revendication 14,
dans lequel
l'ADNc de la sous-unité alpha de l'inhibine aviaire contient les séquences SEQ ID n°1 ou SEQ ID n°3.

16. Produit de gène de fusion qui encode un gène pour l'expression de la sous-unité alpha de la protéine inhibine aviaire ou un fragment de celle-ci fusionnée à un gène encodé pour l'expression d'une protéine vecteur.

17. Produit de gène de fusion selon la revendication 16,
dans lequel
le gène encodé pour l'expression de la sous-unité alpha de la protéine inhibine aviaire ou le fragment de celle-ci est encodé pour la production d'une protéine inhibine ou d'un fragment de celle-ci, qui inclut la séquence d'amino-acides exposée dans SEQ ID n°2 ou SEQ ID n°4.

18. Produit de gène de fusion selon la revendication 16,
qui inclut la séquence de SEQ ID n°1 ou SEQ ID n°3.

19. Produit de gène de fusion selon l'une quelconque des revendications 16 à 18,
dans lequel
le gène encodé pour l'expression de la protéine vecteur est encodé pour la production d'une protéine choisie parmi la protéine laint le maltose, la termalbumine bovine, l'ovalbumine, la flagelline, l'hermocyamine de patelle à trou central, l'albumine térique, la gamma globuline ou des polymères d'amino acides.

20. Vecteur comprenant une séquence d'ADN selon le gène de fusion selon l'une quelconque des revendications 16 à 19.

21. Cellule contenant le vecteur selon la revendication 20.
